# EUROPEAN PATENT SPECIFICATION

(11) **EP 0 944 592 B1**
(45) Date of publication and mention of the grant of the patent: **26.01.2005**
(21) Application number: 97945802.3
(22) Date of filing: 28.11.1997
(51) Int. Cl.: C07C 401/00, A61K 31/59

(54) **VITAMIN D3 DERIVATIVES**
VITAMIN D3-DERIVATE
DERIVES DE VITAMINE D3

(30) Priority: 04.12.1996 GB 9625271
(43) Date of publication of application: 29.09.1999
(73) Proprietor: LEO PHARMA A/S (with secondary name: LEO PHARMACEUTICAL PRODUCTS LTD. A/S), 2750 Ballerup (DK)
(72) Inventor: VON DAEHNE, Welf, DK-2960 Rungsted Kyst (DK)
(74) Representative: Kinzebach, Werner, Dr.
(86) International application number: PCT/DK1997/000546
(87) International publication number: WO 1998/024762

(56) References cited:
- EP-A- 0 398 217
- EP-A- 0 580 968
- EP-A- 0 646 576
- EP-A- 0 654 467
- EP-A- 0 796 843
- WO-A-97/19058
- US-A- 5 087 619

## Description

This invention relates to a hitherto unknown class of compounds which shows strong activity in inducing differentiation and inhibiting undesirable proliferation of certain cells, including cancer cells and skin cells, as well as antiinflammatory and immunomodulating effects, to pharmaceutical preparations containing these compounds, to dosage units of such preparations, and to their use in the treatment and prophylaxis of hyperparathyroidism, particularly secondary hyperparathyroidism associated with renal failure, of diseases characterized by abnormal cell differentiation and/or cell proliferation such as cancer, leukemia, myelofibrosis, and psoriasis, of a number of disease states including diabetes mellitus, hypertension, acne, alopecia, skin ageing, AIDS, neurodegenerative disorders such as Alzheimer's disease, host versus graft reactions, rejection of transplants, inflammatory diseases such as rheumatoid arthritis and asthma, for prevention and/or treatment of steroid induced skin atrophy, and for promoting osteogenesis and treating osteoporosis.

The compounds of the present invention are represented by the general formula I 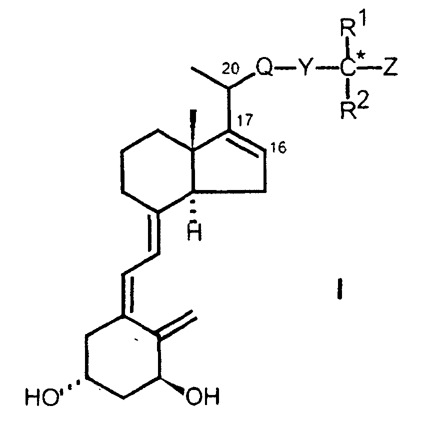 in which formula Q is a C₁-C₆ hydrocarbylene diradical; Y is either a single bond, a carbonyl group or a methylene, ethylene, -CH(OH)-, -O-(C₆H₄)- (ortho, meta, para) or -S-(C₆H₄)- (ortho, meta, para) diradical; R¹ and R², which may be the same or different, stand for hydrogen or a C₁-C₆ hydrocarbyl radical; or R¹ and R², when taken together with the carbon atom (starred in formula I) bearing the group Z, can form a C₃-C₆ carbocyclic ring; and Z is hydrogen or hydroxy; with the proviso that when, at the same time, Q is ethylene, Y is methylene, R¹ and R² stand for methyl or trifluoromethyl, and Z is hydroxy, or when, at the same time, Q is ethylene, Y stands for carbonyl or -CH(OH)-, R¹ and R² are methyl, and Z is hydroxy, then the configuration at C-20 cannot be R.

In the context of this invention, the expression hydrocarbyl radical (hydrocarbylene diradical) indicates the residue after the removal of 1 (2) hydrogen atom(s) from a straight, branched or cyclic, saturated or unsaturated hydrocarbon.

Examples of Q include, but are not limited to, methylene, ethylene, tri-, tetra- and pentamethylene, -CH=CH-, -CH=CH-CH=CH-, -CH=CH-C≡C-, -CH=CH-CH₂-, -CH₂-(C₆H₄)- (ortho, meta, para), -C≡C-, -C≡C-CH₂-, -CH(R)-(CH₂)₂-, -CH(R)-CH=CH-, and -CH(R)-C≡C- in which R is hydroxy, C₁-C₄ alkoxy or C₁-C₄ alkyl. Compounds of formula I in which Q is -(CH₂)ₙ-, n being an integer from 1 to 4, or stands for a straight carbon chain with one or two, in the latter case conjugated, double or triple bonds or for -CH(R)-C≡C-, R being defined as above, are of particular interest.

Examples of R¹ and R² when taken separately include, but are not limited to, hydrogen, methyl, ethyl, vinyl, normal-, iso- and cyclopropyl, and trifluoromethyl.

Examples of and R¹ and R² when taken together include di- tri-, tetra- and pentamethylene.

The compounds of the invention comprise more than one stereoisomeric form (e.g., R or S configuration at C-20; E or Z configuration when a double bond is present in the group Q). The invention covers all these stereoisomers in pure form as well as mixture thereof.

In addition, the compounds of the invention in which one or more of the hydroxy groups are masked as groups which can be reconverted to hydroxy groups *in vivo* are prodrugs of I.

Compounds of formula I in which Z is hydrogen also may act as prodrugs, as these compounds are relatively inactive *in vitro,* but are converted to active compounds of formula I by enzymatic hydroxylation after administration to the patient.

It has been shown that 1α,25-dihydroxy-vitamin D₃ (1,25(OH)₂D₃) influences the effects and/or production of interleukins (Muller, K. et al., *Immunol*. *Lett.,* 17, 361-366 (1988)), indicating the potential use of this compound in the treatment of diseases characterized by a dysfunction of the immune system, e.g. autoimmune diseases, AIDS, host versus graft reactions, and rejection of transplants or other conditions characterized by an abnormal interleukin-1 production, e.g. inflammatory diseases such as rheumatoid arthritis and asthma.

It has also been shown that 1,25(OH)₂D₃ is able to stimulate the differentiation of cells and inhibit excessive cell proliferation (Abe, E. et al., *Proc. Natl*. *Acad. Sci*., U.S.A., 78, 4990-4994 (1981)), and it has been suggested that this compound might be useful in the treatment of diseases characterized by abnormal cell proliferation and/or cell differentiation such as leukemia, myelofibrosis and psoriasis.

Also, the use of 1,25(OH)₂D₃, or its pro-drug 1α-OH-D₃ , for the treatment of hypertension (Lind, L. et al., *Acta Med. Scand*., 222, 423-427 (1987)) and diabetes mellitus (Inomata, S. et al., *Bone Mineral.,* 1, 187-192 (1986)) has been suggested. Another indication for 1,25(OH)₂D₃ is suggested by the recent observation of an association between hereditary vitamin D resistance and alopecia: treatment with 1,25(OH)₂D₃ may promote hair growth (Editorial, Lancet, March 4, p. 478 (1989)). Also, the fact that topical application of 1,25(OH)₂D₃ reduces the size of sebaceous glands in the ears of male Syrian hamsters suggests that this compound might be useful for the treatment of acne (Malloy V.L. et al., The Tricontinental Meeting for Investigative Dermatology, Washington, (1989)).

However, the therapeutic possibilities in such indications are severely limited by the well known potent effect of 1,25(OH)₂D₃ on calcium metabolism; elevated blood concentrations will rapidly give rise to hypercalcemia. Thus, this compound and some of its potent synthetic analogues are not completely satisfactory for use as drugs in the treatment of e.g. psoriasis, leukemia or immune diseases which may require continuous administration of the drug in relatively high doses.

A number of vitamin D analogues have recently been described that show some degree of selectivity in favour of the cell differentiation inducing/cell proliferation inhibiting activity *in vitro* as compared with the effects on calcium metabolism *in vivo* (as measured in increased serum calcium concentration and/or increased urinary calcium excretion), which adversely limit the dosage that can safely be administered. One of the first of these to appear, calcipotriol (INN) or calcipotriene (USAN), has been developed on the basis of this selectivity and is now recognized worldwide as an effective and safe drug for the topical treatment of psoriasis.

A study with another analogue (EB 1089) selected on this basis supports the concept that systemically administered vitamin D analogues may inhibit breast cancer cell proliferation *in vivo* at sub-toxic doses (Colston, K.W. et al., *Biochem*. *Pharmacol*. 44, 2273-2280 (1992) and Mathiasen, I.S. et al., *J*. *Steroid Biochem*. *Molec. Biol*., 46, 365-371 (1993)).

Promising immunosuppressive activities of vitamin D analogues have been reviewed (Binderup, L., *Biochem*. *Pharmacol*. 43, 1885-1892 (1992)). Thus, a series of 20-epi-vitamin D analogues has been identified as potent inhibitors of T-lymphocyte activation *in vitro* (Binderup, L. et al, *Biochem*. *Pharmacol*. 42, 1569-1575 (1991)). Two of these analogues, MC 1288 and KH 1060, systemically administered, have shown immunosuppressive activities *in vivo* in experimental animal models. Additive or synergistic effects were observed in combination with low-dose cyclosporin A. KH 1060, alone or in combination with cyclosporin A, has also been shown to prevent autoimmune destruction of transplanted islets in diabetic NOD mice (Bouillon, R. et al. In: *Vitamin D, a Pluripotent Steroid Hormone: Structural Studies, Molecular Endocrinology and Clinical Applications;* Norman, A.W., Bouillon, R., Thomasset, M., Eds.; de Gruyter, Berlin, 1994, pp. 531-552). MC 1288 was able to prolong survival of cardiac and small bowel grafts in rats (Johnsson, C. et al. In: *Vitamin D, a Pluripotent Steroid Hormone: Structural Studies, Molecular Endocrinology and Clinical Applications;* Norman, A.W., Bouillon, R., Thomasset, M., Eds.; de Gruyter, Berlin, 1994, pp. 549-550). However, in all these studies, the dosages of the analogues that produced significant immunosuppression also induced increases in serum calcium levels. There is therefore a continuing need for new analogues with an acceptable combination of prolonged therapeutic activity and minimum toxic effects.

The compounds of the present invention provide a hitherto undisclosed series of 16-dehydro-1α,25-dihydroxy-vitamin D₃ analogues with potent immunosuppressive and cell proliferation inhibitory activities. The compounds of formula I are characterized by the presence of a 16,17-double bond in the five-membered ring D, and their absolute configuration at C-20 can be either R or S.

Analogues of vitamin D having a 16,17-double bond in ring D are not new. For example, Hoffmann-La Roche Inc. in US Patents No. 5,087,619/1992 and No. 5,145,846/1992 disclose the synthesis and use of 25-hydroxy- and 1α,25-di-hydroxy-16-ene-cholecalciferols, their 23-ene and 23-yne analogues as well as corresponding 26,26,26,27,27,27-hexafluoro derivatives. Uskokovic, M.R. et al. describe the synthesis and biological activities of 16-ene analogues of 1,25-di-hydroxycholecalciferol (In: *Vitamin D: Gene Regulation*, *Structure-Function Analysis and Clinical Application*; Norman, A.W., Bouillon, R., Thomasset, M., Eds.; de Gruyter, Berlin, 1991, pp. 139-145). Hoffmann-La Roche A.G. in European Patent Application No. 0580968/1993 disclose the synthesis and use of 26,26,26,27,27,27-hexafluoro analogues of 25-hydroxy-, 1α,25-dihydroxy-, and 1a-fluoro-25-hydroxy-16-ene-23-yne-cholecalciferols and their corresponding 19-nor derivatives. McLane, J.A. et al. describe stable and active metabolites of 1,25-dihydroxy-16-ene-cholecalciferol (US Patent No. 5,401,733/1995). However, it should be noted that these and other prior art 16-dehydro-vitamin D₃ compounds are characterized by the presence of the natural vitamin D configuration of the C-20 methyl group. Furthermore, all possess as the rest of the side chain (the other substituent on C-20) the aliphatic six-carbon skeleton of the natural vitamin D₃ side chain. Finally, these compounds contain optionally a 23,24-double or triple bond.

The compounds of the present invention differ from the prior art 16-dehydro-vitamin D₃ analogues in the skeleton of the C-20 side chain which is not restricted to being either aliphatic or six-carbon, and in the location of the optional double or triple bond(s) which is/are not restricted to being between carbon atoms 23 and 24. In addition, the configuration at C-20 can be either R (the natural vitamin D configuration) or S.

In order to demonstrate the effectiveness of the compounds of. formula I, the information of Table A is referred to: "HaCaT, rel.", "MLR, rel.", and "Calc., rel."; the meaning of which is explained in the following.

A useful assay for the rating of test compounds for antiproliferative activity in skin cells, e.g. antipsoriatic effect, is the *in vitro* assay using HaCaT, a spontaneously immortalized, non-tumorigenic human skin keratinocyte cell line (Mørk Hansen, C. et al., *J*. *Invest*. *Dermatol*. 1, 44-48 (1996)), measuring ³H -thymidine uptake.

An *in vitro* assay for the rating of test compounds for immunosuppressive potency is the mixed lymphocyte reaction assay, "MLR", measuring the allogeneic stimulation of mouse spleen lymphocytes: lymphocytes, obtained from the spleens of BALB/c and CB6F1 mice, are stimulated by co-cultivating 5x10⁶/ml cells from BALB/c mice (responders) with 7.5x10⁶/ml cells from CB6F1 mice (inducers). The mixed cultures of lymphocytes are incubated with the test compounds for 72 hours. Cellular DNA-synthesis is assessed by the incorporation of ³H-thymidine in the DNA.

Generally, the classical effects of 1,25(OH)₂D₃ on the calcium balance in the organism, including calcemic and calciuric activities, are unwanted in the vitamin D analogues of the present invention, in which selectivity for e.g. inhibition of the proliferation of certain cells and/or immunosuppressive activity is normally desired.

The calcemic activity of the compounds was determined in rats *in vivo,* as previously described (Binderup, L., Bramm, E., *Biochem. Pharmacol*. 37, 889-895 (1988)). In Table A, column "Calc., rel,", the calcemic activities of selected compounds (relative to 1,25(OH)₂D₃) are listed; as mentioned, low values for the compounds of the present invention are ordinarily preferred.

It appears from Table A that the selected Compounds 107 and 111 are considerably more potent than 1,25(OH)₂D₃ in the HaCaT-assay (psoriasis model), while the calcernic activity is similar to that of 1,25(OH)₂D₃.

Concerning the other important property of the compounds of the invention, their immunosuppressive activity, it is apparent from Table A, column "MLR, rel.", that the selected Compounds 107 and 111Have potent effects.

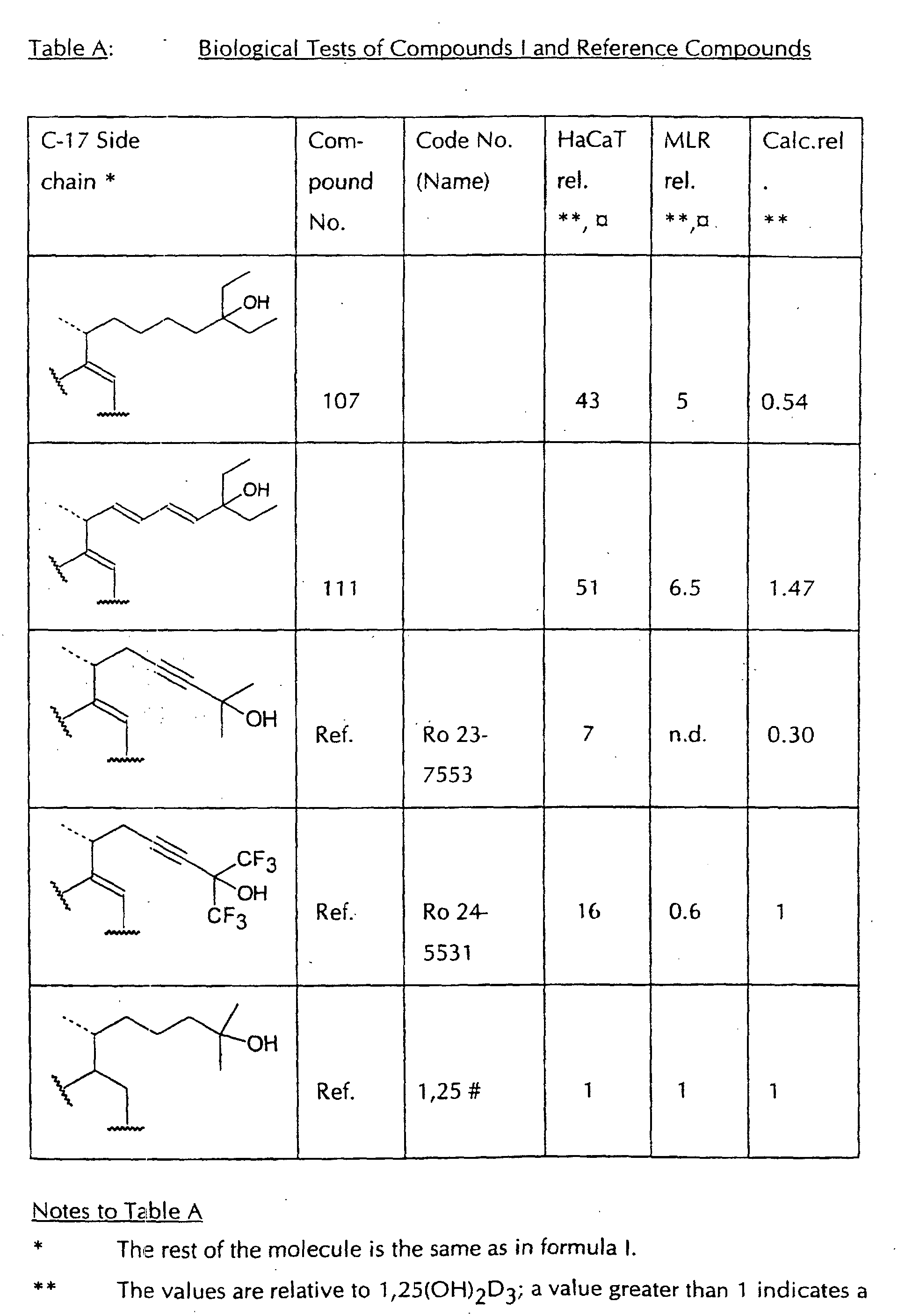

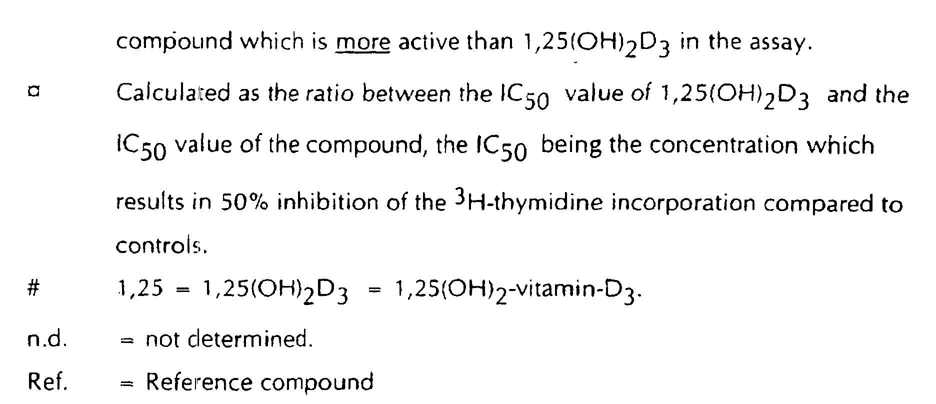

The compounds of formula I may be prepared from the C-20 epimeric alcohols 3 and 4, a synthesis of which from the vitamin D-derived aldehyde 1 (Calverley, M.J., *Tetrahedron,* 43, 4609-4619 (1987)) via the 20-keto compound 2 has been reported (Hansen, K. et al. In: *Vitamin D: Gene Regulation, Structure-Function Analysis and Clinical Application;* Norman, A.W., Bouillon, R., Thomasset, M., Eds.; de Gruyter, Berlin, 1991, pp. 161-162), for example by the general methods of Schemes 1 and 2.

More specifically, the preparation of the 16-dehydro tosylates 13/14 and the analogous aldehydes 15/16, important intermediates in the synthesis of the compounds of formula I from said starting materials, is outlined in Scheme 1, whereas the further conversion of the above key intermediates to the compounds of formula I is outlined in Scheme 2.

The synthesis of the side chain building blocks V to VIII, or analogues thereof, may be performed by standard procedures described in the literature/International Patent Applications Nos. WO 87/00834, WO 89/10351, WO 91/00271, WO 91/00855, WO 91/15475, WO 93/19044, and WO 95/02577.

The following standard abbreviations are used throughout this disclosure:DMF = N,N-dimethylformamide; DMSO = dimethyl sulfoxide; Et =ethyl; Hal = halogen; "HF" = 5% hydrogen fluoride in acetonitrile:water (7:1, v/v); Me = methyl; Ph = phenyl; PPTS = pyridinium p-toluenesulfonate; TBAF = tetra-n-butylammonium fluoride trihydrate; TBDMS = tert-butyldimethylsilyl; THF = tetrahydrofuran; THP = tetrahydro-4H-pyran-2-yl; TMS = trimethylsilyl; Ts = p-toluenesulfonyl (tosyl). 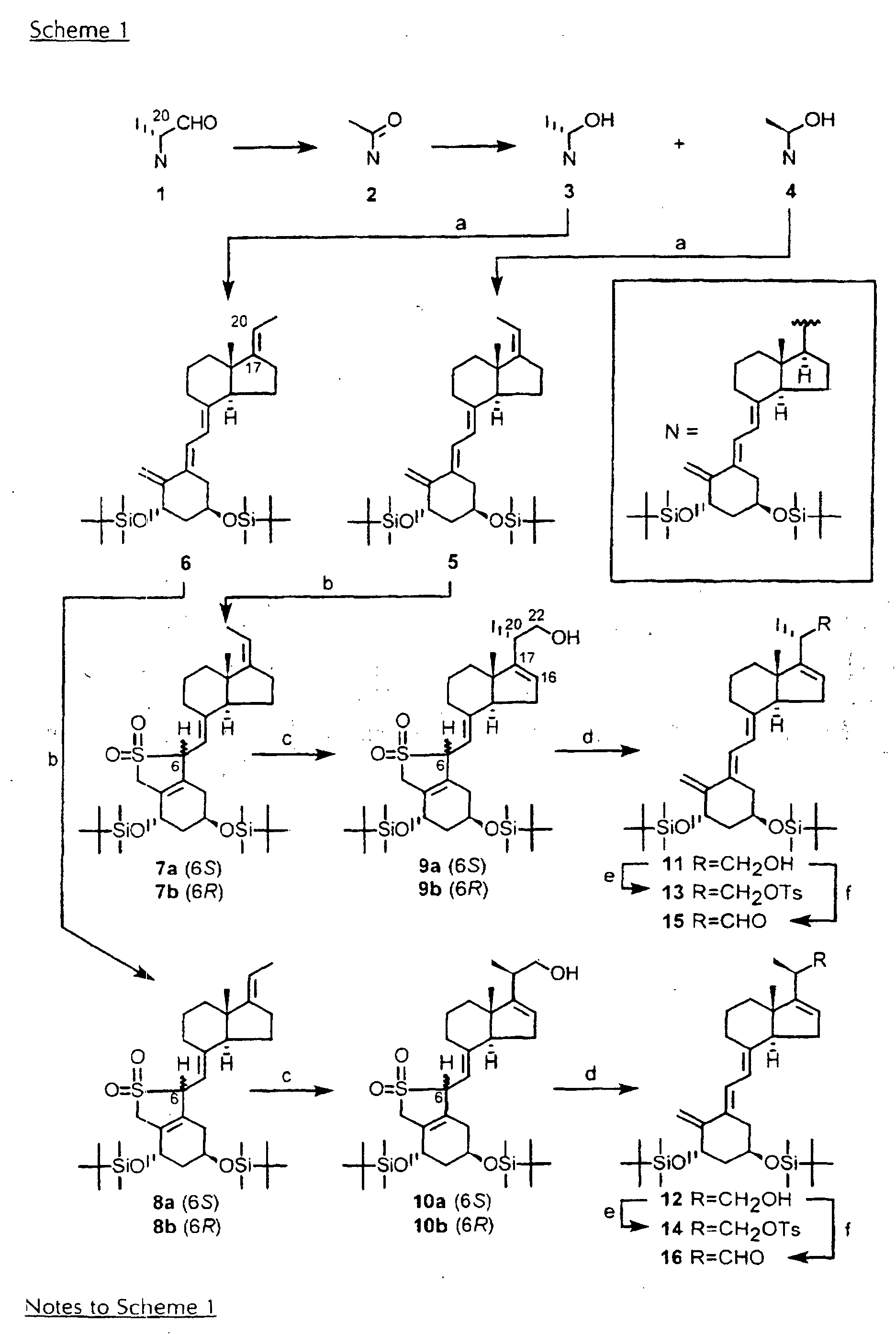 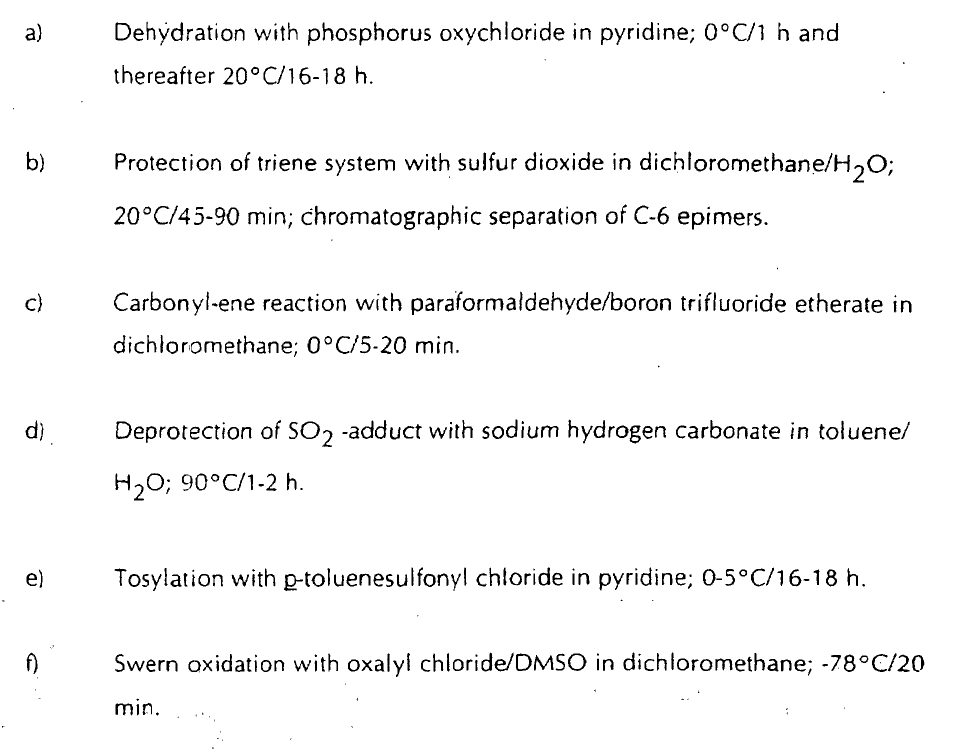 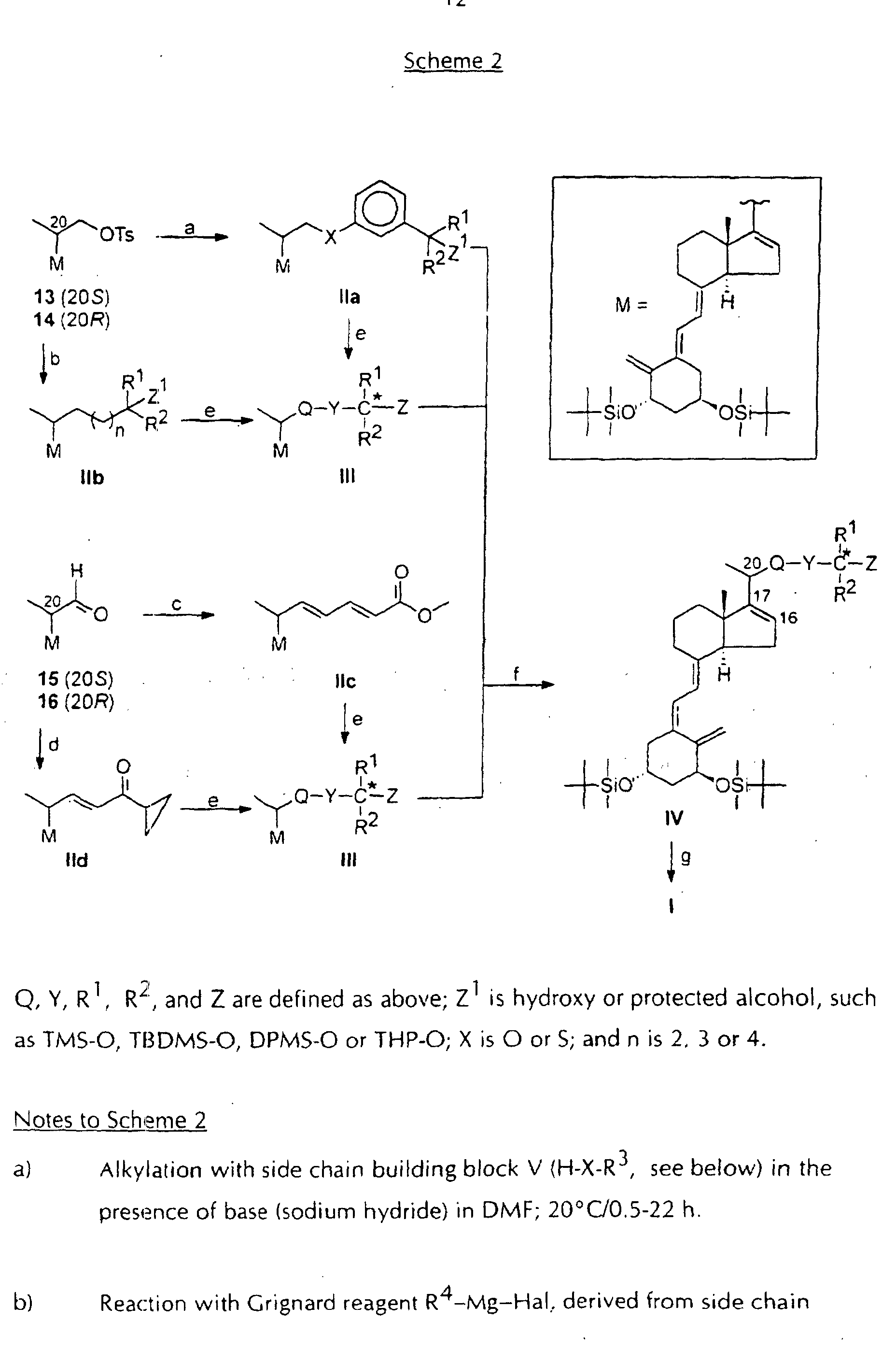 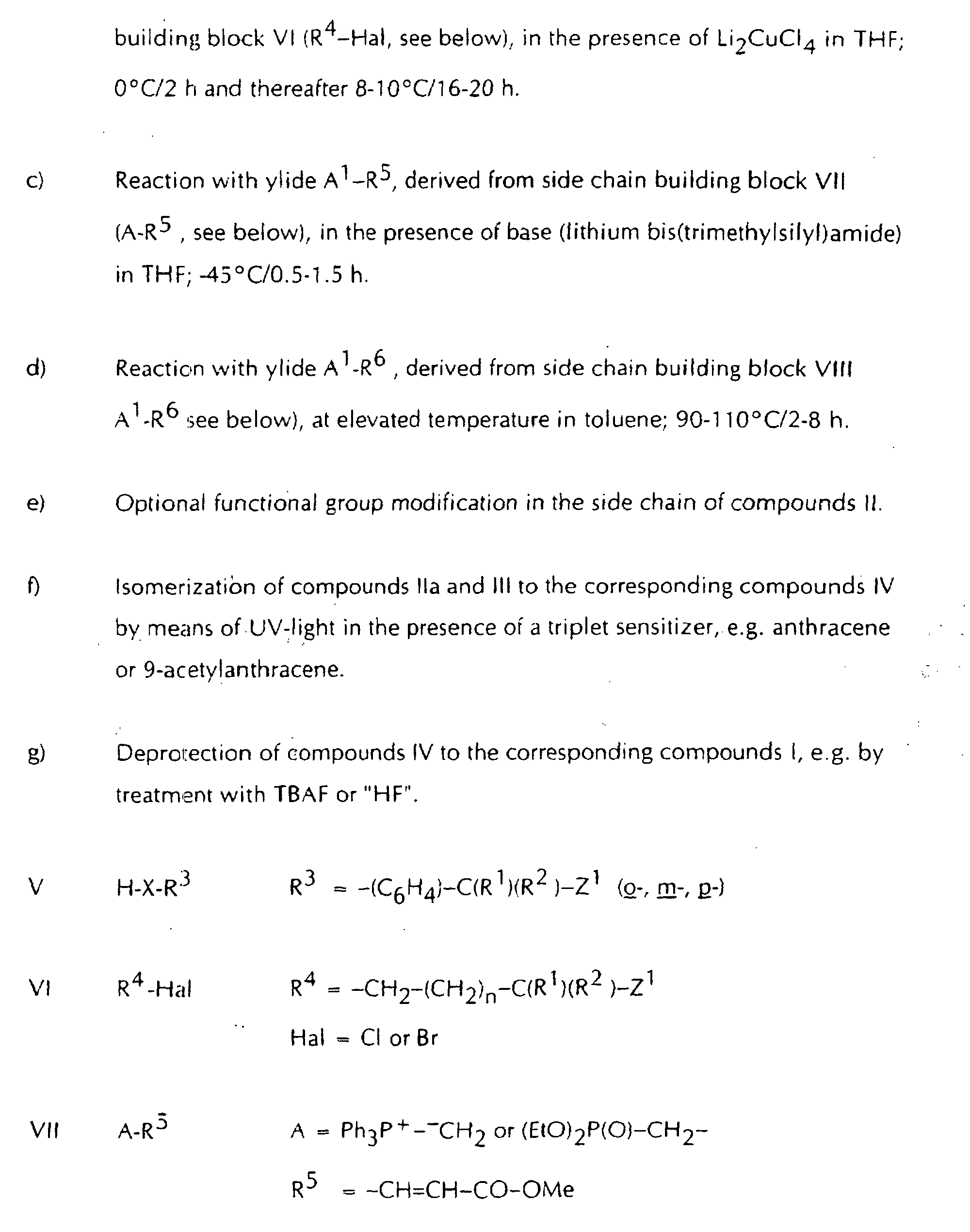 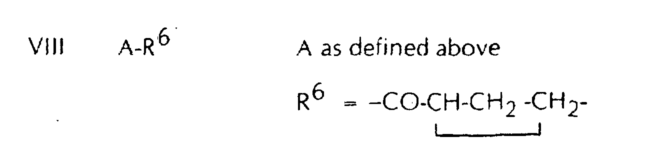 In A¹-R⁵ and A¹-R⁶, A¹ stands for Ph₃P⁺-⁻CH - or (EtO)₂P(O)-CHLi-, R⁵ and R⁶ are as defined above.

The present compounds are intended for use in pharmaceutical compositions which are useful in the local or systemic treatment of human and veterinary disorders as described above.

The present compounds may be used in combination with other pharmaceuticals or treatment modalities. In the treatment of psoriasis the present compounds may be used in combination with e.g. steroids or with other treatments e.g. light- or UV-light-treatment or the combined PUVA-treatment. In the treatment of cancer the present compounds may be used in combination with other anti-cancer drugs or anti-cancer treatments, such as radiation treatment. In the prevention of graft rejection and graft versus host reaction, or in the treatment of autoimmune diseases, the present compounds may advantageously be used in combination with other immunosuppressive/immunoregulating drugs or treatments, e.g. with cyclosporin A.

The amount required of a compound of formula I (hereinafter referred to as the active ingredient) for therapeutic effect will, of course, vary both with the particular compound, the route of administration and the mammal under treatment. The compounds of the invention can be administered by the parenteral, intra-articular, enteral or topical routes. They are well absorbed when given enterally and this is the preferred route of administration in the treatment of systemic disorders. In the treatment of dermatological disorders like psoriasis or eye diseases topical or enteral forms are preferred.

While it is possible for an active ingredient to be administered alone as the raw chemical, it is preferable to present it as a pharmaceutical formulation. Conveniently, the active ingredient comprises from 0.1 ppm to 0.1% by weight of the formulation.

The formulations, both for veterinary and for human medical use, of the present invention thus comprise an active ingredient in association with a pharmaceutically acceptable carrier therefore and optionally other therapeutic ingredient(s). The carrier(s) must be "acceptable" in the sense of being compatible with the other ingredients of the formulations and not deleterious to the recipient thereof.

The formulations include e.g. those in a form suitable for oral, ophthalmic, rectal, parenteral (including subcutaneous, intramuscular and intravenous), transdermal, intra-articular and topical, nasal or buccal administration.

By the term "dosage unit" is meant a unitary, i.e. a single dose which is capable of being administered to a patient, and which may be readily handled and packed, remaining as a physically and chemically stable unit dose comprising either the active material as such or a mixture of it with solid or liquid pharmaceutical diluents or carriiers.

The formulations may conveniently be presented in dosage unit form and may be prepared by any of the methods well known in the art of pharmacy. All methods include the step of bringing the active ingredient into association with the carrier which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. The active ingredient may also be administered in the form of a bolus, electuary or paste.

Formulations for rectal administration may be in the form of a suppository incorporating the active ingredient and a carrier, or in the form of an enema.

Formulations suitable for parenteral administration conveniently comprise a sterile oily or aqueous preparation of the active ingredient which is preferably isotonic with the blood of the recipient. Transdermal formulations may be in the form of a plaster.

Formulations suitable for intra-articular or ophthalmic administration may be in the form of a sterile aqueous preparation of the active ingredient which may be in microcrystalline form, for example, in the form of an aqueous microcrystalline suspension. Liposomal formulations or biodegradable polymer systems may also be used to present the active ingredient for both intra-articular and ophthalmic administration.

Formulations suitable for topical or ophthalmic administration include liquid or semi-liquid preparations such as liniments, lotions, gels, applicants, oil-in-water or water-in-oil emulsions such as creams, ointments or pastes; or solutions or suspensions such as drops.

Formulations suitable for administration to the nose or buccal cavity include powder, self-propelling and spray formulations, such as aerosols and atomizers.

In addition to the aforementioned ingredients, the formulations of this invention may include one or more additional ingredients, such as diluents, binders, preservatives etc.

The compositions may further contain other therapeutically active compounds usually applied in the treatment of the above mentioned pathological conditions, such as other immunosuppressants in the treatment of immunological diseases, or steroids in the treatment of dermatological diseases.

The present invention further concerns a method for treating patients suffering from one of the above pathological conditions, said method consisting of administering to a patient in need of freatment an effective amount of one or more compounds of formula I, alone or in combination with one or more other therapeutically active compounds usually applied in the treatment of said pathological conditions. The treatment with the present compounds and/or with further therapeutically active compounds may be simultaneous or with intervals.

In the systemic treatment daily doses of from 0.001-2 µg per kilogram bodyweight, preferably from 0.002-0.3 µg/kg of mammal bodyweight, for example 0.003-0.3 µg/kg of a compound of formula I are administered, typically corresponding to a daily dose for an adult human of from 0.2 to 25 µg. In the topical treatment of dermatological disorders, ointments, creams or lotions containing from 0.1-500 µg/g, and preferably from 0.1-100 µg/g, of a compound of formula I are administered. For topical use in ophthalmology ointments, drops or gels containing from 0.1-500 µg/g, and preferably from 0.1-100 µg/g, of a compound of formula I are administered. The oral compositions are formulated, preferably as tablets, capsules, or drops, containing from 0.05-50 µg, preferably from 0.1-25 µg, of a compound of formula I, per dosage unit.

The invention will now be further described in the following

### General Procedures, Preparations and Examples

### General

The exemplified compounds I are listed in Table 5, whereas intermediates 5-16 and compounds of the general formulas II, III and IV are listed in Tables 1-4.

For nuclear magnetic resonance spectra (300 MHz) chemical shift values (δ) are quoted for deuteriochloroform solutions relative to internal tetramethylsilane (δ = 0) or chloroform (δ = 7.25). The value for a multiplet, either defined (doublet (d), triplet (t), quartet (q)) or not (m) at the approximate mid point is given unless a range is quoted (s = singlet, b = broad). Coupling constants (1) are given in Hertz (Hz), and are sometimes approximated to the nearest unit.

Ether is diethyl ether, and was dried over sodium. THF was dried over sodium-benzophenone. Petroleum ether refers to the pentane fraction. Reactions were routinely run under an argon atmosphere at room temperature unless otherwise noted. The work-up procedure referred to involves dilution with the specified solvent (otherwise the organic reaction solvent), extraction with water and then brine, drying over anhydrous MgSO₄, and concentration in vacuo to give a residue. Chromatography was performed on silica gel.

**Table 1**

| Intermediates of Formulas 5-16 (Scheme 1) | | |
|---|---|---|
| Compound No. | Preparation No. | General Procedure |
| 5 | 1 | 1 |
| 7a | 3 | 2 |
| 7b | 4 | 2 |
| 9a | 7 | 3 |
| 9b | 8 | 3 |
| 11 | 11 | 4 |
| 13 | 13 | 5 |
| 15 | 15 | 6 |
| 6 | 2 | 1 |
| 8a | 5 | 2 |
| 8b | 6 | 2 |
| 10a | 9 | 3 |
| 10b | 10 | 3 |
| 12 | 12 | 4 |
| 14 | 14 | 5 |
| 16 | 16 | 6 |

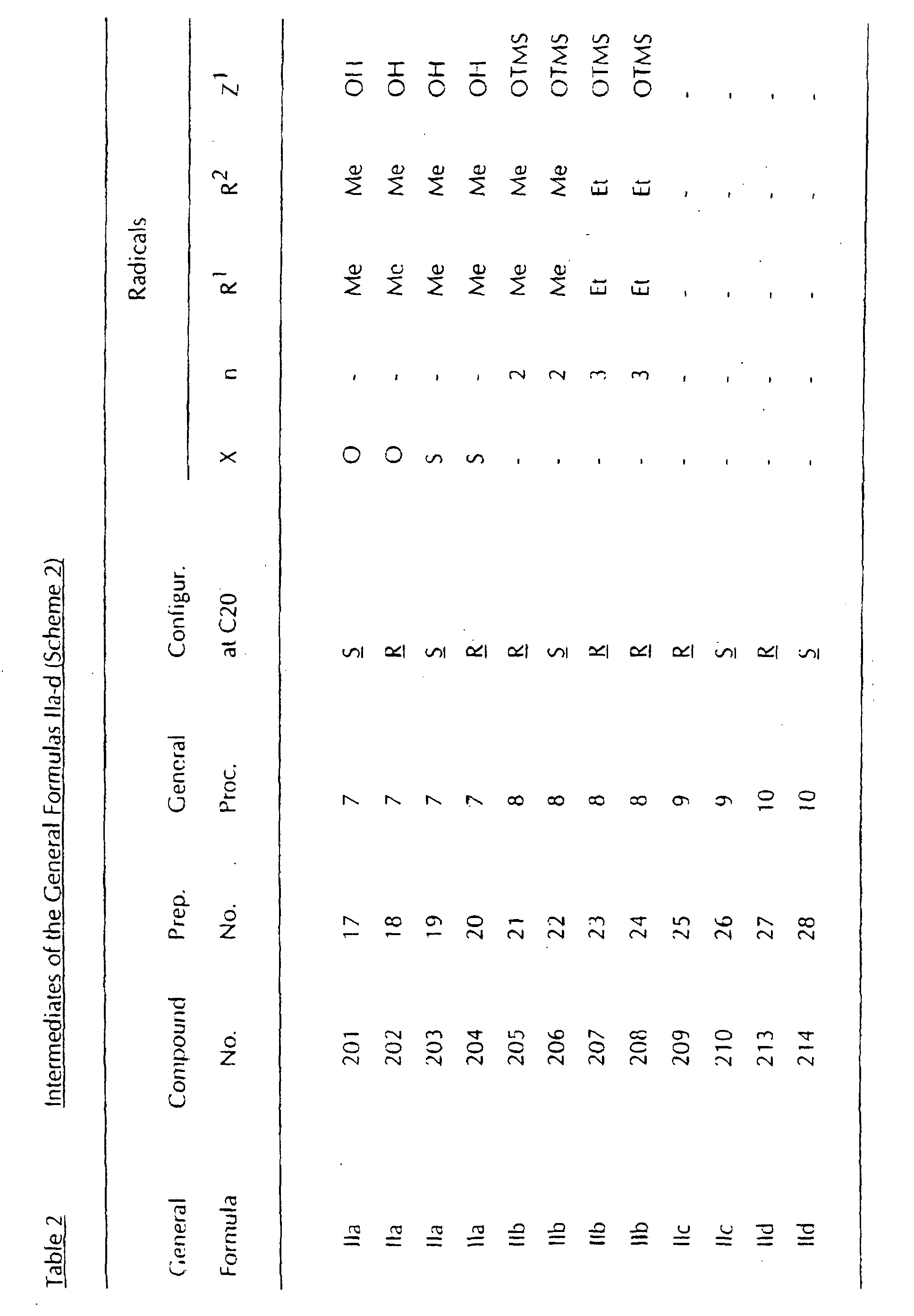

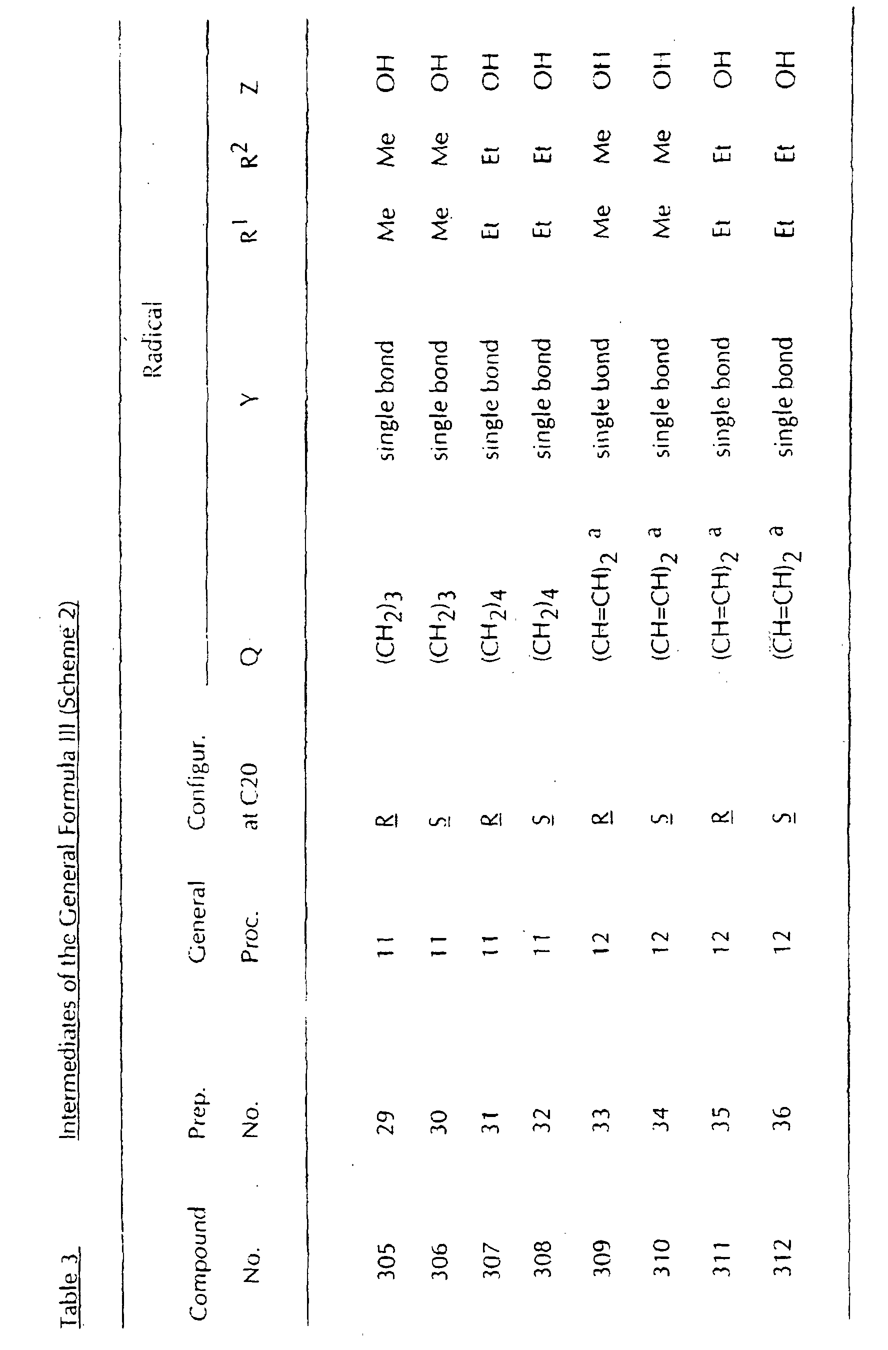

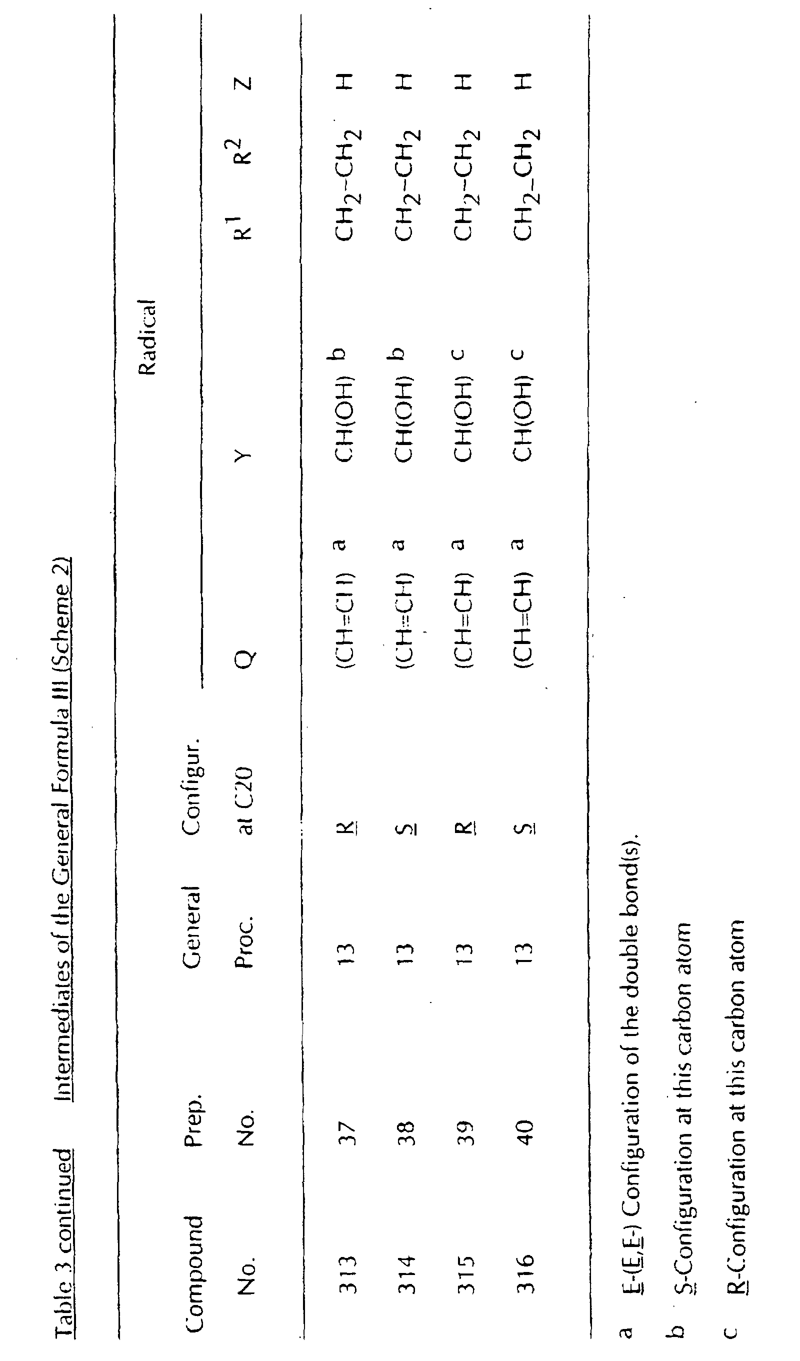

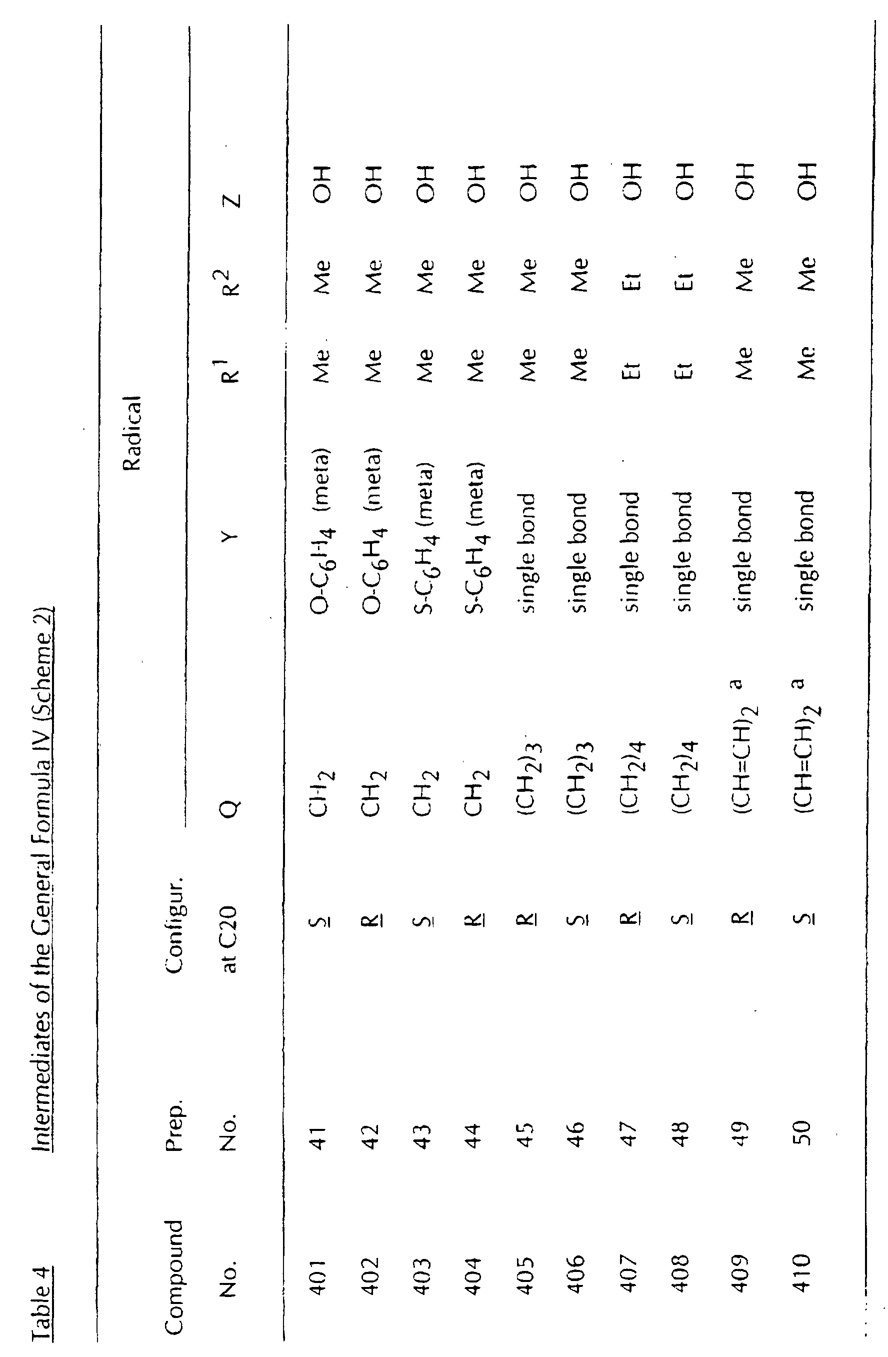

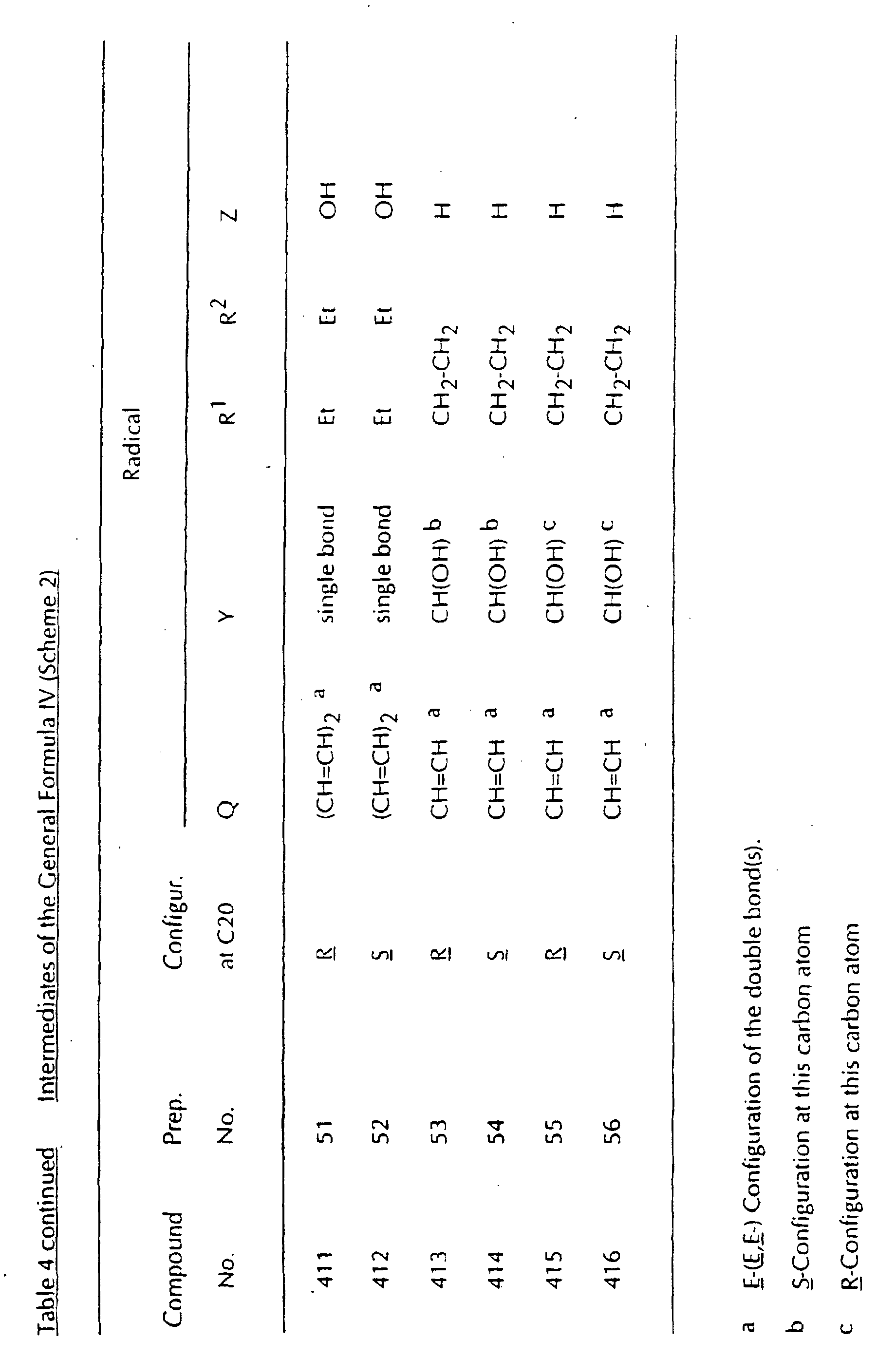

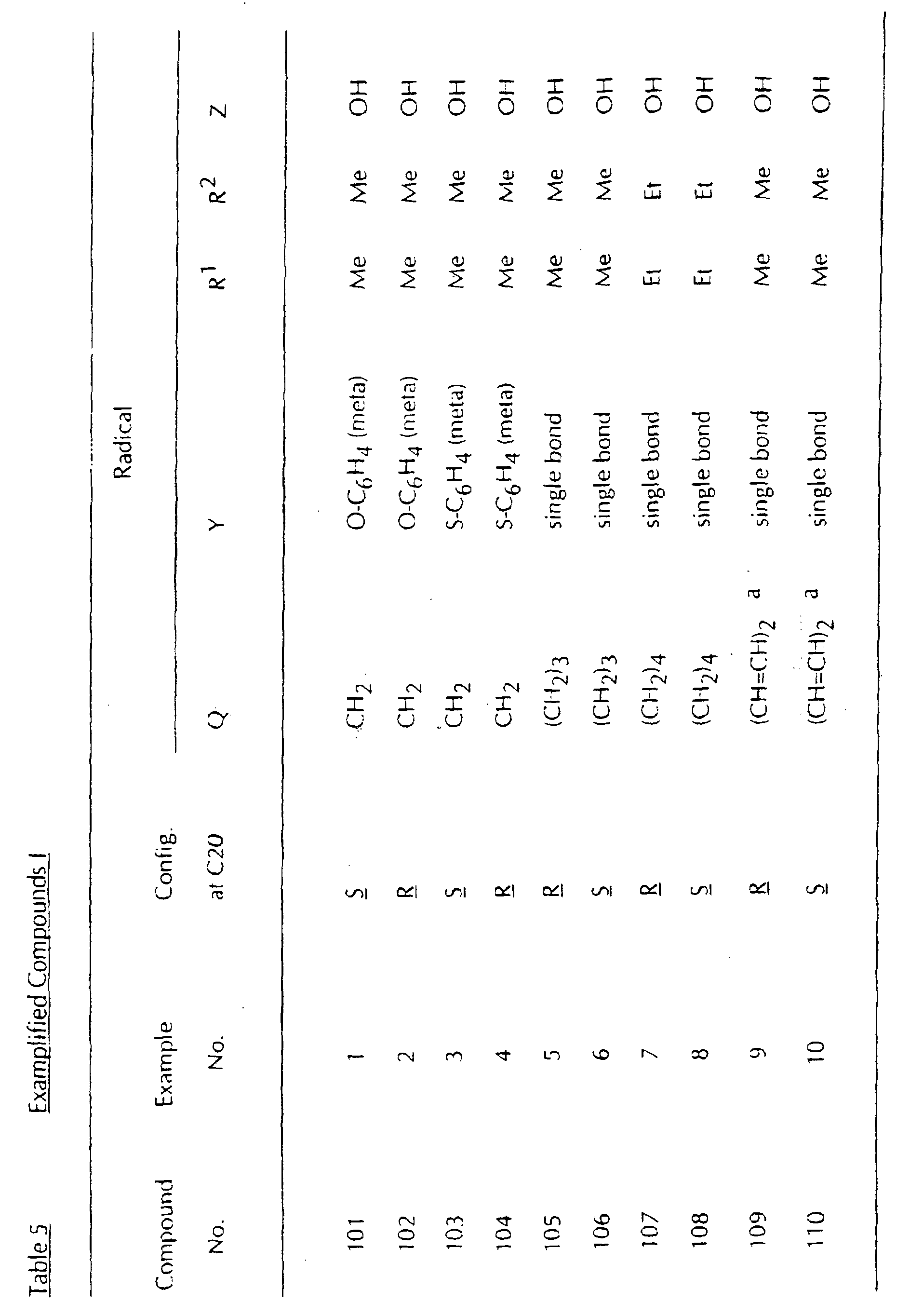

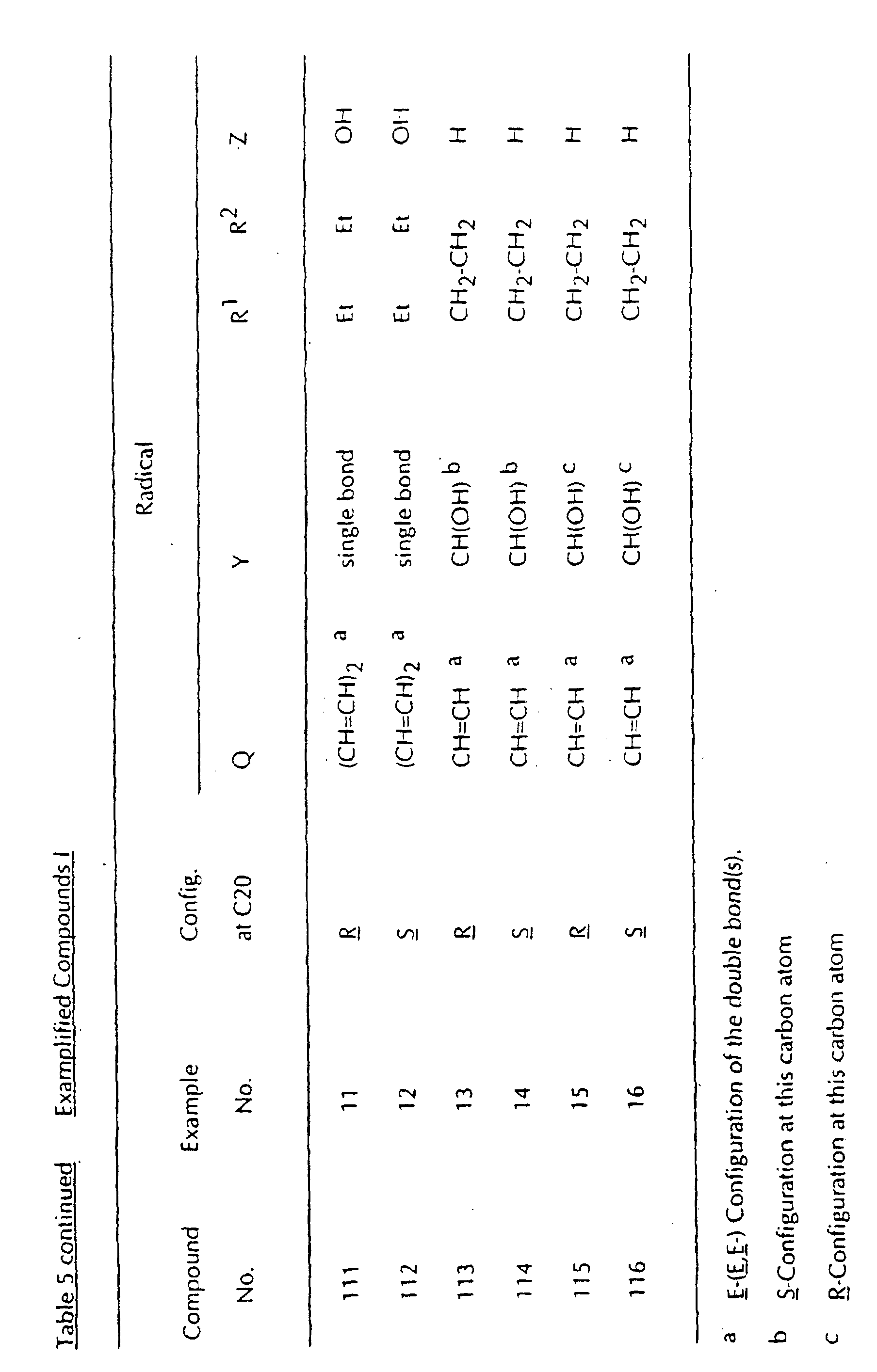

### General Procedures

### General Procedure 1: Dehydration of compounds 4 and 3 to give the corresponding anhydro compounds 5 and 6 (Preparations 1-2)

A solution of compound 4 (or 3) (2.81 g, 5 mmol) in pyridine (40 ml) was cooled to 0°C, and phosphorus oxychloride (4.6 ml, 50 mmol) was added dropwise over 5 minutes with stirring. After stirring for one hour at the low temperature and 16 hours at ambient temperature, the reaction mixture was poured onto ice-cooled ethyl acetate (120 ml), and water (40 ml) was carefully added while stirring. An apparent pH of 2.8 in the mixture was adjusted by addition of 4 N hydrochloric acid (80 ml) and the phases were separated. After an additional extraction of the aqueous layer with ethyl acetate (60 ml), the combined organic extracts were worked up. The crude product was either used for the next step without further purification (compound 5) or purified by crystallization from ether-MeOH (compound 6).

### General Procedure 2: Reaction of compounds 5 and 6 with sulfur dioxide to give the corresponding SO₂ adducts 7a/7b and 8a/8b (Preparations 3-6)

To a solution of crude compound 5 (or pure 6) (5 mmol) in dichloromethane (25 ml) and water (10 ml) was added an ice-cooled, ca. 1.5 M solution of sulfur dioxide in dichloromethane (100 ml) with vigorous stirring. The mixture was stirred for 45 minutes at ambient temperature and then poured onto ice-water (100 ml). The apparent pH of the mixture was adjusted to 5.6 with 2 N sodium hydroxide (62 ml). The organic phase was separated and, after an additional extraction of the aqueous layer (dichloromethane, 25 ml), worked up. The residue was chromatographed (eluent: 5% to 10% ether in petroleum ether) to separate the 6(S) and 6(R) SO₂-adducts 7a and 7b (or 9a and 9b).

### General Procedure 3: Ene-reaction of SO₂-adducts 7a, 7b, 8a and 8b with paraformaldehyde to the corresponding SO₂-protected 16-dehydro alcohols 9a, 9b, 10a and 10b (Preparations 7-10)

To a solution of compound 7a (or 7b, 8a, 8b) (2,42 g, 4 mmol) in dichloromethane (80 ml) was added at 0°C with stirring paraformaldehyde (0.60 g, 20 mmol) followed by boron trifluoride etherate (0.10 ml, 0.4 equiv). After stirring at 0°C for 15 minutes, the reaction was quenched with 1/15 M phosphate buffer (pH 6.5) (60 ml), and the mixture was worked up. The residue was chromatographed (eluent: 10% to 15% ethyl acetate in petroleum ether) to separate minor amounts of the less polar starting material from the more polar title compound (9a or 9b, 10a, 10b).

### General Procedure 4: Deprotection of the SO₂-adducts 9a/9b and 10a/10b to give the corresponding 16-dehydro alcohols 11 and 12 (Preparations 11-12)

To a solution of compound 9a (or 9b) (1.27 g, 2 mmol) in toluene (20 ml) was added water (10 ml) and sodium hydrogen carbonate (0.67 g, 8 mmol), and the mixture was stirred at 85-90°C for 1.5 hours. After cooling to room temperature, the reaction mixture was worked up (toluene) to give compound 11 as a foam.

Similar treatment of compound 10a (or 10b) afforded compound 12 as an amorphous powder.

### General Procedure 5: Tosylation of the 16-dehydro alcohols 11 and 12 to yield the corresponding tosylates 13 and 14 (Preparations 13-14)

To a stirred solution of compound 11 (or 12) (2 mmol) in pyridine (10 ml) was added at 0°C p-toluenesulfonyl chloride (0.76 g, 4 mmol), and the mixture was stirred for 2 hours at 0-5°C and then left in the refrigerator overnight (16 hours). The reaction mixture was poured onto an ice-cooled mixture of ethyl acetate (40 ml) and water, and the apparent pH in the aqueous layer was adjusted to 2.6 with 4 N hydrochloric acid. After separation of the aqueous phase and an additional extraction with ethyl acetate, the combined organic extracts were worked up. The residue was purified by chromatography (eluent: 5% ether in petroleum ether) to give pure 13 (or 14).

### General Procedure 6: Oxydation of the 16-dehydro alcohols 11 and 12 to the corresponding aldehydes 15 and 16 (Swern oxydation) (Preparations 15-16)

A stirred solution of oxalyl cnloride (0.45 ml, 5 mmol) in dry dichloromethane was cooled to -78°C, and 2 N dimethyl sulfoxide in dry dichloromethane (5.6 ml, 11.2 mmol) was added by syringe. After stirring for 10 minutes at the low temperature, a solution of 11 (or 12) (4 mmol) in dry dichformethane (12 ml) was added (syringe). Stirring was continued for another 20 minutes before the reaction was quenched by addition of triethylamine (2.1 ml, 15 mmol). The cooling bath was removed, and the reaction mixture was allowed to warm to room temperature (about 45 minutes with stirring) and worked up (dichloromethane) to give crude compound 15 (or 16) which was used in the following step without further purification.

### Ceneral Procedure 7: Alkylation of compounds 13 and 14 to give compounds IIa (Preparations 17-20)

To a solution of the appropriate alkylating agent HXR³ (1.5 mmol) in dry DMF (10 ml) was added sodium hydride (2.25 mmol), and the mixture was stirred for 20 minutes. A solution of compound 13 (or 14) in dry DMF (5 ml) was then added by syringe, and stirring was continued for either 30-40 minutes (S-alkylation) or 16-22 hours (O-alkylation). After cooling to 0-5°C, excess reagent was decomposed by dropwise addition of water (0.5-1.0 ml), and the reaction mixture was worked up (ethyl acetate). The residue was purified by chromatography (eluent: 5%-10% ether in petroleum ether) to afford the corresponding compound IIa.

### General Procedure 8: Reaction of compounds 13 and 14 with Grignard reagents R⁴MgHal, derived from side chain building blocks II (R⁴Hal) to give compounds IIb (Preparations 21-24)

To magnesium turnings (220 mg, 1.1 atomic equivalents) in a dry flask was added dropwise with stirring in an argon atmosphere a solution of the appropriate compound VI (8.25 mmol) in dry THF (7.5 ml). Stirring was continued under heating to reflux for 45 minutes. The stirred Grignard reagent was treated at 0°C with a solution of lithium chloride (32 mg) and anhydrous cupric chloride (50 mg) in dry THF (5 ml) followed after 15 minutes by a solution of compound 13 (or 14) in dry THF (5 ml). After stirring for 18 hours at 5-10°C, the reaction mixture was worked up (ether). The crude product was purified by chromatography to yield the desired compound IIb.

### General Procedure 9: Reaction of aldehydes 15 and 16 with ylides A¹R⁵ derived from side chain building block VII (AR⁵), to give compounds IIc (Preparations 25-26)

To a stirred solution of aldehyde 15 (or 16) (1.0 mmol) and the appropriate compound VII (1.8 mmol) in dry THF (5 ml) was added dropwise via a syringe at -50°C 1 M lithium bis(trimethylsilyl)amide in dry THF (1.5 ml). Stirring at the low temperature was continued for another hour before the reaction mixture was allowed to warm to -10°C (15-20 minutes). It was quenched with a few drops of water and worked up (ether). The residue was purified by chromatography to give the desired compound IIc.

### General Procedure 10: Reaction of aldehydes 15 and 16 with ylides A¹R⁶, derived from side chain building block VIII (A¹R⁶), to give compounds IId (Preparations 27-28)

To a solution of aldehyde 15 (or 16) (1.8 mmol) in dry toluene (20 ml) was added the appropriate compound VIII (3.6 mml), and the mixture was stirred at 90-110°C for 2-4 hours. After cooling to 0°C, the mixture was filtered, and the filtrate concentrated and purified by chromatography to give the desired compound IId.

### General Procedure 11: Deprotection of compounds IIb to the corresponding compounds III with PPTS (Preparations 29-32)

To a solution of the appropriate compound IIb (0.5 mmol) in THF (3 ml) and ethanol (6 ml) was added PPTS (15 mg) and the mixture was stirred for one hour. Work up (ethyl acetate) yielded a crude product which was purified by chromatography to give the desired compound III.

### General Procedure 12: Reaction of compound IIc with alkyl lithium to give the corresponding compounds III (Preparations 33-36)

Pre-cooled (-15°C) alkyl lithium in ether (3-4 molar equivalents) was added dropwise at -78°C via a syringe to a stirred solution of the appropriate compound IIc (0.5 mmol) in dry THF (6 ml). After a further 45 minutes at -78°C, the reaction was quenched with a few drops of water, warmed to 20°C and worked up (ether). The residue was chromatographed to yield the desired compound III.

### General Procedure 13: Reduction of compounds IId to the corresponding compounds III (Preparations 37-40)

To stirred solution of the appropriate compound IId (0.8 mmol) in THF (4 ml) was added at 0°C 0.4 M Ce Cl₃ • 7 H₂O in ethanol (2 ml) followed by sodium borohydride (76 mg, 2 mmol). Methanol (4 ml) was added over 10 minutes with stirring, and after a further 20 minutes the mixture was worked up (ethyl acetate). The residue was purified by chromatography to give the desired compound III.

### General Procedure 14: Isomerization of compounds IIa and III to the corresponding compounds IV (Preparations 41-56)

A solution of the appropriate compound IIa or III (0.28 mmol), anthracene (0.10 g, 0.56 mmol) and triethylamine (0.20 ml, 1.4 mmol) in dichloromethane (16 ml) in a 25 ml round-bottomed Pyrex flask was irradiated with UV-light from a high pressure ultraviolet lamp, type TQ760Z2 (Hanau), at ca. 10°C for 30 minutes while stirring. The reaction mixture was evaporated in vacuo, and the residue was treated with petroleum ether (2x2 ml) and filtered. The filtrate was concentrated and purified by chromatography to afford the title compound.

### General Procedure 15: Deprotection of compounds IV to the corresponding compounds I by treatment with "HF" (Examples 1-8 and 13-16)

To a stirred solution of the appropriate compound IV (0.25 mmol) in ethyl acetate (1.5 ml) was added acetonitrile (6 ml) followed by a 5% solution of hydrofluoric acid in acetonitrile-H₂O 7:1 (2.0 ml). After stirring for a further 45-60 minutes, 1 M potassium hydrogen carbonate (10 ml) was added, and the reaction mixture was worked up (ethyl acetate). The residue was purified by chromatography (eluant: 30% pentane in ethyl acetate) to give the desired compound I.

### General Procedure 16: Deprotection of compounds IV to the corresponding compounds I by treatment with tetra-n-butylammonium fluoride (Examples 9-12)

To a solution of the appropriate compound IV (0.18 mmol) in THF (4.5 ml) was added TBAF trihydrate (0.29 g, 0.9 mmol), and the mixture was heated to reflux for one hour with stirring. After addition of 0.2 M sodium hydrogen carbonate (5 ml), the mixture was worked up (ethyl acetate). The residue was purified by chromatography (eluant: 50% ethyl acetate in pentane) to yield the title compound.

### Preparations

### Preparation 1: Compound 5

Method: General Procedure 1
Starting material: Compound 4
¹H NMR δ 0.06 (m, 12H), 0.76 (s, 3H), 0.85 (s, 9H), 0.89 (s, 9H), 1.66 (m, 3H), 1.45-2.50 (m, 13H), 2.56 (dd, 1H), 2.86 (m, 1H), 4.22 (m, 1H), 4.53 (m, 1H), 4.94 (m, 1H), 4.98 (m, 1H), 5.19 (m, 1H), 5.85 (d, 1H), 6.44 (d, 1H).

### Preparation 2: Compound 6

Method: General Procedure 1
Starting Material: Compound 3
Mp. 87-88°C

| | | |
|---|---|---|
| Anal. | Calcd. for C₃₃H₅₈O₂Si₂ | C 73.00, H 10.77 |
| | Found | C 72.90, H 10.82 |

¹H NMR δ 0.06 (m, 12H), 0.62 (s, 3H), 0.86 (s, 9H), 0.90 (s, 9H), 1.56 (d, 3H), 1.20-2.10 (m, 10H), 2.32 (m, 3H), 2.57 (dd, 1H), 2.89 (m, 1H), 4.22 (m, 1H), 4.53 (m, 1H), 4.94 (m, 1H), 4.99 (m, 1H), 5.08 (m, 1H), 5.86 (d, 1H), 6.45 (d, 1H).

### Preparation 3: Compound 7a

Method: General Procedure 2
Starting material: Compound 5
Mp. 117-118°C

| | | |
|---|---|---|
| Anal. | Calcd. for C₃₃H₅₈O₄SSi₂ | C 65.29, H 9.63, S 5.28 |
| | Found | C 65.12, H 9.54, S 5.22 |

¹H NMR δ 0.05 (m, 12H), 0.86 (s, 9H), 0.87 (s, 9H), 0.87 (s, 3H), 1.66 (m, 3H), 1.40-2.50 (m, 14H), 2.60 (m, 1H), 3.60 (bd, 1H), 3.93 (m, 1H), 4.19 (m, 1H), 4.36 (m, 1H), 4.64 (d, 1H), 4.74 (d, 1H), 5.17 (m, 1H).

### Preparation 4: Compound 7b

Method: General Procedure 2
Starting material: Compound 5
¹H NMR δ 0.06 (m, 12H), 0.78 (s, 3H), 0.87 (s, 9H), 0.88 (s, 9H), 1.66 (m, 3H), 1.45-2.45 (m, 14H), 2.56 (m, 1H), 3.63 (bd, 1H), 3.92 (bd, 1H), 4.15 (m, 1H), 4.38 (m, 1H), 4.62 (d, 1H), 4.84 (d, 1H), 5.19 (m, 1H).

### Preparation 5: Compound 8a

Method: General Procedure 2
Starting material: Compound 6
Mp. 117-118°C

| | | |
|---|---|---|
| Anal. | Calcd. for C₃₃H₅₈O₄SSi₂ | C 65.29, H 9.63, S 5.28 |
| | Found | C 65.14, H 9.54, S 5.08 |

¹H NMR δ 0.06 (m, 12H), 0.72 (s, 3H), 0.87 (s, 9H), 0.88 (s, 9H), 1.36 (m, 1H), 1.55 (d, 3H), 1.45-2.05 (m, 10H), 2.10-2.45 (m, 3H), 2.63 (m, 1H), 3.60 (bd, 1H), 3.93 (m, 1H), 4.18 (m, H), 4.36 (m, 1H), 4.65 (d, 1H), 4.75 (d, 1H), 5.10 (m, 1H).

### Preparation 6: Compound 8b

Method: General Procedure 2
Starting material: Compound 6
¹H NMR δ 0.06 (m, 12H), 0.64 (s, 3H), 0.86 (s, 9H), 0.88 (s, 9H), 1.55 (d, 3H), 1.20-1.95 (m, 10H), 2.08 (dd, 1H), 2.30 (m, 3H), 2.60 (m, 1H), 3.63 (bd, 1H), 3.92 (bd, 1H), 4.16 (m, 1H), 4.38 (m, 1H), 4.62 (d, 1H), 4.85 (d, 1H), 5.10 (m, 1H).

### Preparation 7 Compound 9a

Method: General Procedure 3
Starting material: Compound 7a
Mp. 115-116°C

| | | |
|---|---|---|
| Anal. | Calcd. for C₃₄H₆₀O₅SSi₂ | C 64.10, H 9.49, S 5.03 |
| | Found | C 63.78, H 9.55, S 5.00 |

¹H NMR δ 0.06 (m, 12H), 0.82 (s, 3H), 0.87 (s, 9H), 0.88 (s, 9H), 1.06 (d, 3H), 1.35-2.50 (m, 14H), 2.61 (m, 1H), 3.57 (m, 3H), 3.93 (m, 1H), 4.18 (m, 1H), 4.37 (m, 1H), 4.63 (d, 1H), 4.82 (d, 1H), 5.43 (m, 1H).

### Preparation 8: Compound 9b

Method: General Procedure 3
Starting material: Compound 7b
Mp. 131-132°C

| | | |
|---|---|---|
| Anal. | Calcd. for C₃₄H₆₀O₅SSi₂ | C 64.10, H 9.49 |
| | Found | C 64.18, H 9.46 |

¹H NMR δ 0.05 (m, 12H), 0.72 (s, 3H), 0.85 (s, 9H), 0.87 (s, 9H), 1.06 (d, 3H), 1.35-2.02 (m, 9H), 2.10-2.65 (m, 6H), 3.58 (m, 3H), 3.92 (bd, 1H), 4.15 (m, 1H), 4.38 (m, 1H), 4.62 (d, 1H), 4.92 (d, 1H), 5.45 (m, 1H).

### Preparation 9: Compound 10a

Method: General Procedure 3
Starting material: Compound 8a
Mp. 125-126°C

| | | |
|---|---|---|
| Anal. | Calcd. for C₃₄H₆₀O₅SSi₂ | C 64.10, H 9.49, S 5.03 |
| | Found | C 63.98, H 9.46, S 4.82 |

¹H NMR δ 0.06 (m, 12H), 0.81 (s, 3H), 0.87 (s, 9H), 0.88 (s, 9H), 1.11 (d, 3H), 1.40-2.50 (m, 14H), 2.60 (m, 1H), 3.47 (dd, 1H), 3.58 (dd, 1H), 3.60 (bd, 1H), 3.93 (m, 1H), 4.19 (m, 1H), 4.37 (m, 1H), 4.64 (m, 1H), 4.82 (m, 1H), 5.45 (m, 1H).

### Preparation 10: Compound 10b

Method: General Procedure 3
Starting material: Compound 8b
Mp. 1 29-130°C
¹H NMR δ 0.06 (m, 12H), 0.72 (s, 3H), 0.86 (s, 9H), 0.88 (s, 9H), 1.11 (d, 3H), 1.35-2.00 (m, 9H), 2.10-2.50 (m, 5H), 2.57 (dd, 1H), 3.46 (dd, 1H), 3.59 (dd, 1H), 3.64 (bd, 1H), 3.92 (bd, 1H), 4.16 (m, 1H), 4.38 (m, 1H), 4.62 (d, 1H), 4.93 (d, 1H), 5.48 (m, 1H).

### Preparation 11: Compound 11

Method: General Procedure 4
Starting material: Compound 9a or 9b

| | | |
|---|---|---|
| Anal. | Calcd. for C₃₄H₆₀O₃SSi₂ | C 71.27, H 10.55 |
| | Found | C 71.00, H 10.59 |

¹H NMR δ 0.05 (m, 12H), 0.71 (s, 3H), 0.85 (s, 9H), 0.90 (s, 9H), 1.06 (d, 3H), 1.35-2.00 (m, 8H), 2.09 (m, 1H), 2.28 (m, 2H), 2.41 (m. 2H), 2.58 (dd, 1H), 2.87 (m, 1H), 3.55 (m, 2H), 4.22 (m, 1H), 4.54 (m, 1H), 4.95 (m, 1H), 4.99 (m, 1H), 5.46 (m, 1H), 5.92 (d, 1H), 6.45 (d, 1H).

### Preparation 12: Compound 12

Method: General Procedure 4
Starting material: Compound 10a or 10b
¹H NMR δ 0.06 (m, 12H), 0.70 (s, 3H), 0.85 (s, 9H), 0.90 (s, 9H), 1.11 (d, 3H), 1.40-1.90 (rn, 7H), 1.93 (m, 1H), 2.10 (m, 1H), 2.20-2.50 (m, 4H), 2.58 (dd, 1H), 2.86 (m, 1H), 3.48 (m, 1H), 3.57 (m, 1H), 4.22 (m, 1H), 4.54 (m, 1H), 4.95 (m, 1H), 4.99 (m, 1H), 5.48 (m, 1H), 5.92 (d, 1H), 6.45 (d, 1H).

### Preparation 13: Compound 13

Method: General Procedure 5
Starting material: Compound 11
Mp. 77-78°C

| | | |
|---|---|---|
| Anal. | Calcd. for C₄₁H₆₆O₅SSi₂ | C 67.72, H 9.15, S 4.41 |
| | Found | C 67.50, H 9.07, S 4.67 |

¹H NMR δ 0.06 (m, 12H), 0.60 (s, 3H), 0.86 (s, 9H), 0.89 (s, 9H), 1.04 (d, 3H), 1.30-1.85 (m, 6H), 1.91 (m, 1H), 2.02 (m, 1H), 2.10-2.38 (m, 3H), 2.44 (s, 3H), 2.40-2.62 (m, 2H), 2.84 (m, 1H), 3.84 (t, 1H), 4.04 (dd. 1H), 4.22 (m, 1H), 4.53 (m, 1H), 4.94 (m, 1H), 4.98 (m, 1H), 5.31 (m, 1H), 5.88 (d, 1H), 6.43 (d, 1H), 7.33 (d, 2H), 7.78 (d, 2H).

### Preparation 14: Compound 14

Method: General Procedure 5
Starting material: Compound 12
Mp. 89-90°C

| | | |
|---|---|---|
| Anal. | Calcd. for C₄₁H₆₆O₅SSi₂ | C 67.72, H 9.15 |
| | Found | C 67.73, H 9.36 |

¹H NMR δ 0.06 (m, 12H), 0.63 (s, 3H), 0.85 (s, 9H), 0.90 (s, 9H), 1.09 (d, 3H), 2.44 (s, 3H), 2.56 (dd, 1H), 0.75-2.50 (m, 12H), 2.83 (m, 1H), 3.73 (t, 1H), 4.03 (dd, 1H), 4.22 (m, 1H), 4.53 (m, 1H), 4.95 (m, 1H), 4.98 (m, 1H), 5.37 (m, 1H), 5.88 (d, 1H), 6.42 (d, 1H), 7.33 (d, 2H), 7.78 (d, 2H).

### Preparation 15: Compound 15

Method: General Procedure 6
Starting material: Compound 11
¹H NMR δ 0.06 (m, 12H), 0.69 (s, 3H), 0.85 (s, 9H), 0.90 (s, 9H), 1.21 (d, 3H), 1.40-2.00 (m, 7H), 2.14 (m, 1H), 2.31 (m, 2H), 2.44 (dd, 1H), 2.58 (m, 1H), 2.87 (m, 1H), 3.05 (m, 1H), 4.22 (m, 1H), 4.53 (m, 1H), 4.95 (m, 1H), 4.99 (m, 1H), 5.52 (m, 1H), 5.92 (d, 1H), 6.44 (d, 1H), 9.45 (d, 1H).

### Preparation 16: Compound 16

Method: General Procedure 6
Starting material: Compound 12
¹H NMR δ 0.06 (m, 12H), 0.70 (s, 3H), 0.85 (s, 9H), 0.89 (s, 9H), 1.24 (d, 3H), 1.00-2.00 (m, 7H), 2.14 (m, 1H), 2.29 (m, 2H), 2.46 (m, 1H), 2.57 (dd, 1H), 2.86 (m, 1H), 3.05 (m, 1H), 4.22 (m, 1H), 4.53 (m, 1H), 4.94 (m, 1H), 4.99 (m, 1H), 5.56 (m, 1H), 5.92 (d, 1H), 6.44 (d, 1H), 9.45 (d, 1H).

### Preparation 17 Compound 201

Method: General Procedure 7
Starting material: Compound 13
Alkylating agent: 3-(1-hydroxy-1-methyl)ethylphenol
Chromatography eluant: 10% ether in pentane
¹H NMR δ 0.05 (m, 12H), 0.72 (s, 3H), 0.85 (s, 9H), 0.90 (s, 9H), 1.20 (d, 3H), 1.57 (s, 6H), 1.15-2.00 (m, 8H), 2.09 (m, 1H), 2.28 (m, 2H), 2.43 (m, 1H), 2.60 (m, 2H), 2.87 (m, 1H), 3.81 (t, 1H), 4.00 (dd, 1H), 4.23 (m, 1H), 4.53 (m, 1H), 4.95 (m, 1H), 4.99 (m, 1H), 5.48 (m, 1H), 5.93 (d, 1H), 6.46 (d, 1H), 6.76 (m, 1H), 7.03 (m, 2H), 7.24 (t, 1H).

### Preparation 18 Compound 202

Method: General Procedure 7
Starting material: Compound 14
Alkylating agent: 3-(1-hydroxy-1-methyl)ethylphenol
Chromatography eluant: 10% ether in pentane
¹H NMR δ 0.05 (m, 12H), 0.72 (s, 3H), 0.85 (s, 9H), 0.90 (s, 9H), 1.23 (d, 3H), 1.57 (s, 6H), 1.40-2.00 (m, 8H), 2.09 (m, 1H), 2.29 (m, 2H), 2.42 (m, 1H), 2.60 (m, 2H), 2.87 (m, 1H), 3.67 (t, 1H), 4.01 (dd, 1H), 4.23 (m, 1H), 4.54 (m, 1H), 4.95 (m, 1H), 4.99 (m, 1H), 5.51 (m, 1H), 5.93 (d, 1H), 6.46 (d, 1H), 6.78 (m, 1H), 7.04 (m, 2H), 7.24 (t, 1H).

### Preparation 19 Compound 203

Method: General Procedure 17
Starting material: Compound 13
Alkylating agent: 3-(1-hydroxy-1-methyl)ethylthiophenol
Chromatography eluant: 10% ether in pentane
¹H NMR δ 0.06 (m, 12H), 0.70 (s, 3H), 0.86 (s, 9H), 0.90 (s, 9H), 1.19 (d, 3H), 1.56 (s, 6H), 1.15-1.87 (m, 7H), 1.92 (m, 1H), 2.07 (m, 1H), 2.20-2.50 (m, 4H), 2.57 (dd, 1H), 2.85 (d, 1H), 2.86 (dd, 1H), 3.20 (dd, 1H), 4.22 (m, 1H), 4.53 (m, 1H), 4.94 (m, 1H), 4.99 (m, 1H), 5.44 (m, 1H), 5.91 (d, 1H), 6.45 (d, 1H), 7.15-7.30 (m, 3H), 7.46 (m, 1H).

### Preparation 20 Compound 204

Method: General Procedure 7
Starting material: Compound 14
Alkylating agent: 3-(1-hydroxy-1-methyl)ethylthiophenol
Chromatography eluant: 10% ether in pentane
   ¹H NMR δ 0.05 (m, 12H), 0.67 (s, 3H), 0.85 (s, 9H), 0.90 (s, 9H), 1.22 (d, 3H), 1.56 (s, 6H), 1.40-1.87 (m, 7H), 1.93 (m, 1H), 2.08 (m, 1H), 2.25 (m, 2H), 2.40 (m, 2H), 2.58 (dd, 1H), 2.79 (dd, 1H), 2.84 (m, 1H), 3.18 (dd, 1H), 4.22 (m, 1H), 4.53 (m, 1H), 4.95 (m, 1H), 4.99 (m, 1H), 5.47 (m, 1H), 5.90 (d, 1H), 6.44 (d, 1H), 7.15-7.30 (m, 3H), 7.48 (m, 1H).

### Preparation 21 Compound 205

Method: General Procedure 8
Starting material: Compound 13
Alkylating agent: 4-Bromo-2-methyl-2-trimethylsilyloxybutane
Chromatography eluant: 1% ether in pentane
¹H NMR δ 0.07 (m, 21H), 0.68 (s, 3H), 0.85 (s, 9H), 0.90 (s, 9H), 1.02 (d, 3H), 1.18 (s, 6H), 1.10-2.45 (m, 18H), 2.60 (dd, 1H), 2.85 (m, 1H), 4.22 (m, 1H), 4.53 (m, 1H), 4.95 (m, 1H), 4.99 (m, 1H), 5.30 (m, 1H), 5.92 (d, 1H), 6.46 (d, 1H).

### Preparation 22 Compound 206

Method: General Procedure 8
Starting material: Compound 14
Alkylating agent: 4-Bromo-2-methyl-2-trimethylsilyloxybutane
Chromatography eluant: 10% ether in pentane
¹H NMR δ 0.06 (m, 12H), 0.10 (s, 9H), 0.69 (s, 3H), 0.85 (s, 9H), 0.90 (s, 9H), 1.05 (d, 3H), 1.19 (s, 6H), 1.10-2.45 (m, 18H), 2.59 (dd, 1H), 2.85 (m, 1H), 4.22 (m, 1H), 4.53 (m, 1H), 4.94 (m, 1H), 4.99 (m, 1H), 5.33 (m, 1H), 5.92 (d, 1H), 6.46 (d, 1H).

### Preparation 23 Compound 207

Method: General Procedure 8
Starting material: Compound 13
Alkylating agent: 6-Bromo-2-ethyl-3-trimethylsilyloxyhexane
Chromatography eluant: 1 % ether in pentane
¹H NMR δ 0.06 (m, 12H), 0.08 (s, 9H), 0.68 (s, 3H), 0.80 (t, 6H), 0.85 (s, 9H), 0.90 (s, 9H), 1.01 (d, 3H), 1.44 (q, 4H), 0.75-2.45 (m, 20H), 2.58 (dd, 1H), 2.85 (m, 1H), 4.22 (m, 1H), 4.54 (m, 1H), 4.95 (m, 1H), 4.99 (m, 1H), 5.30 (m, 1H), 5.92 (d, 1H), 6.46 (d, 1H).

### Preparation 24 Compound 208

Method: General Procedure 8
Starting material: Compound 14
Alkylating agent: 6-Bromo-3-ethyl-3-trimethylsilyloxyhexane
Chromatography eluant: 1% ether in pentane
¹H NMR δ 0.06 (m, 12H), 0.08 (s, 9H), 0.69 (s, 3H), 0.80 (t, 6H), 0.85 (s, 9H), 0.90 (s, 9H), 1.04 (d, 3H), 1.15-2.45 (m, 20H), 1.44 (q, 4H), 2.59 (dd, 1H), 2.86 (m, 1H), 4.22 (m, 1H), 4.54 (m, 1H), 4.95 (m, 1H), 4.99 (m, 1H), 5.32 (m, 1H), 5.92 (d, 1H), 6.46 (d, 1H).

### Preparation 25 Compound 209

Method: General Procedure 9
Starting material: Compound 15
Chromatography eluant: 2.5% ether in pentane
¹H NMR δ 0.06 (m, 12H), 0.65 (s, 3H), 0.85 (s, 9H), 0.89 (s, 9H), 1.19 (d, 3H), 1.15-1.87 (m, 6H), 1.93 (m, 1H), 2.06 (m, 1H),.2.25 (m, 2H), 2.38 (dd, 1H), 2.58 (dd, 1H), 2.84 (m, 1H), 3.00 (m, 1H), 3.73 (s, 3H), 4.22 (m, 1H), 4.52 (m, 1H), 4.95 (m, 1H), 4.98 (m, 1H), 5.41 (m, 1H), 5.81 (d, 1H, J = 15.4 Hz), 5.90 (d, 1H), 6.03 (dd, 1H, J = 15.2 Hz and 7.8 Hz), 6.16 (dd, 1H), J = 15.2 Hz and 10.5 Hz), 6.44 (d, 1H), 7.27 (dd, 1H, J = 15.4 Hz and 10.5 Hz).

### Preparation 26 Compound 210

Method: General Procedure 9
Starting material: Compound 16
Chromatography eluant: 2.5% ether in pentane
¹H NMR δ 0.06 (m, 12H), 0.69 (s, 3H), 0.85 (s, 9H), 0.89 (s, 9H), 1.20 (d, 3H), 1.35-1.87 (m, 6H), 1.93 (m, 1H), 2.07 (m, 1H), 2.25 (m, 2H), 2.41 (dd, 1H), 2.58 (dd, 1H), 2.84 (bd, 1H), 2.98 (m, 1H), 3.73 (s, 3H), 4.22 (m, 1H), 4.54 (m, 1H), 4.95 (m, 1H), 4.98 (m, 1H), 5.41 (m, 1H), 5.80 (d, 1H, J = 15.4 Hz), 5.91 (d, 1H), 6.11 (m, 2H), 6.44 (d, 1H), 7.27 (dd, 1H, J = 15.4 Hz and 9.9 Hz).

### Preparation 27 Compound 213

Method: General Procedure 10
Starting material: Compound 15
Chromatography eluant: 2.5% ether in petroleum ether
¹H NMR δ 0.05 (m, 12H), 0.67 (s, 3H), 0.85 (s, 9H), 0.90 (s, 9H), 0.80-0.95 (m, 2H), 1.07 (m, 2H), 1.22 (d, 3H), 1.45-2.35 (m, 11H), 2.41 (dd, 1H), 2.58 (dd, 1H), 2.85 (m, 1H), 3.06 (m, 1H), 4.22 (m, 1H), 4.53 (m, 1H), 4.95 (m, 1H), 4.98 (m, 1H), 5.44 (m, 1H), 5.90 (d, 1H), 6.20 (dd, 1H, J = 1.0 and 15.8 Hz), 6.44 (d, 1H), 6.8.2 (dd, 1H, J = 7.9 and 15.8 Hz).

### Preparation 28 Compound 214

Method: General Procedure 10
Starting material: Compound 16
Chromatography eluant: 2.5% ether in petroleum ether
¹H NMR δ 0.05 (m, 12H), 0.70 (s, 3H), 0.85 (s, 9H), 0.90 (s, 9H), 0.82-0.95 (m, 2H), 1.07 (m, 2H), 1.24 (d, 3H), 1.35-2.50 (m, 12H), 2.58 (dd, 1H), 2.84 (bd, 1H), 3.04 (m, 1H), 4.22 (m, 1H), 4.53 (m, 1H), 4.95 (m, 1H), 4.98 (m, 1H), 5.46 (m, 1H), 5.91 (d, 1H), 6.18 (dd, 1H, J = 1.0 and 15.8 Hz), 6.44 (d, 1H), 6.85 (dd, 1H, J = 7.7 and 15.8 Hz).

### Preparation 29 Compound 305

Method: General Procedure 11
Starting material: Compound 205
Chromatography eluant: 10% ether in pentane
¹H NMR δ 0.05 (m, 12H), 0.68 (s, 3H), 0.85 (s, 9H), 0.89 (s, 9H), 1.02 (d, 3H), 1.19 (s, 6H), 1.10-2.45 (m, 19H), 2.58 (dd, 1H), 2.85 (m, 1H), 4.22 (m, 1H), 4.53 (m 1H), 4.94 (m, 1H), 4.98 (m, 1H), 5.31 (m, 1H), 5.92 (d, 1H), 6.45 (d, 1H).

### Preparation 30 Compound 306

Method: General Procedure 11
Starting material: Compound 206
Chromatography eluant: 10% ether in pentane
¹H NMR δ 0.05 (m, 12H), 0.69 (s, 3H), 0.85 (s, 9H), 0.89 (s, 9H), 1.05 (d, 3H), 1.20 (s, 6H), 1.10-2.45 (m, 19H), 2.58 (dd, 1H), 2.85 (m, 1H), 4.22 (m, 1H), 4.54 (m, 1H), 4.94 (m, 1H), 4.99 (m, 1H), 5.33 (m, 1H), 5.91 (d, 1H), 6.45 (d, 1H).

### Preparation 31 Compound 307

Method: General Procedure 11
Starting material: Compound 207
Chromatography eluant: 10% ether in pentane
¹H NMR δ 0.05 (m, 12H), 0.67 (s, 3H), 0.84 (t, 6H), 0.85 (s, 9H), 0.90 (s, 9H), 1.01 (d, 3H), 1.44 (q, 4H), 0.80-2.45 (m, 21H), 2.58 (dd, 1H), 2.85 (m, 1H), 4.22 (m, 1H), 4.53 (m, 1H), 4.94 (m, 1H), 4.98 (m, 1H), 5.29 (m, 1H), 5.91 (d, 1H), 6.45 (d, 1H).

### Preparation 32 Compound 308

Methodl: General Procedure 11
Starting material: Compound 208
Chromatography eluant: 10% ether in pentane
¹H NMR δ 0.05 (m, 12H), 0.68 (s, 3H), 0.85 (s, 6H), 0.85 (t, 9H), 0.90 (s, 9H), 1.04 (d, 3H), 1.45 (q, 4H), 1.15-2.45 (m, 21H), 2.59 (dd, 1H), 2.86 (m 1H), 4.22 (m, 1H), 4.54 (m, 1H), 4.94 (m, 1H), 4.99 (m, 1H), 5.32 (m, 1H), 5.91 (d, 1H), 6.46 (d, 1H).

### Preparation 33 Compound 309

Method: General Procedure 12
Starting material: Compound 209
Organometallic reagent: Methyl lithium
Chromatography eluant: 5% ether in petroleum ether

### Preparation 34 Compound 310

Method: General Procedure 12
Starting material: Compound 210
Organometallic reagent: Methyl lithium
Chrornatography eluant: 2.5% ether in petroleum ether

### Preparation 35 Compound 311

Method: General Procedure 12
Starting material: Compound 209
Orgariometallic reagent: Ethyl lithium
Chromatography eluant: 5% ether in pentane
¹H NMR δ 0.06 (m, 12H), 0.67 (s, 3H), 0.85 (t, 6H), 0.85 (s, 9H), 0.89 (s, 9H), 1.16 (d, 3H), 1.10-1.87 (m, 1H), 1.92 (m, 1H), 2.04 (m, 1H), 2.25 (m, 2H), 2.38 (dd, 1H), 2.59 (dd, 1H), 2.89 (m, 2H), 4.22 (m, 1H), 4.53 (m, 1H), 4.94 (m, 1H), 4.98 (m, 1H), 5.38 (m, 1H), 5.54 (d, 1H, J = 15.3 Hz), 5.59 (dd, 1H, J = 14.9 Hz and 8.0 Hz), 5.90 (d, 1H), 6.02 (dd, 1H, J = 14.9 Hz and 10.3 Hz), 6.17 dd, 1H, J = 15.3 Hz and 10.3 Hz), 6.45 (d, 1H).

### Preparation 36 Compound 312

Method: General Procedure 12
Starting material: Compound 210
Organometallic reagent: Ethyl lithium
Chromatography eluant: 5% ether in pentane
0.06 (m, 12H), 0.69 (s, 3H), 0.85 (s, 9H), 0.86 (t, 6H), 0.90 (s, 9H), 1.17 (d, 3H), 1.25-1.87 (m, 11H), 1.93 (m, 1H), 2.05 (m, 1H), 2.26 (m, 2H), 2.42 (dd, 1H), 2.59 (dd, 1H), 2.86 (m, 2H), 4.22 (m, 1H), 4.54 (m, 1H), 4.95 (m, 1H), 4.99 (m, 1H), 5.38 (m, 1H), 5.54 (d, 1H, J = 15.3 Hz), 5.64 (dd, 1H, J = 15.0 Hz and 7.7 Hz), 5.91 (d, 1H), 6.01 (dd, 1H, J = 15.0 Hz and 10.3 Hz), 6.18 (dd, 1H, J = 15.3 Hz and 10.3 Hz), 6.45 (d, 1H).

### Preparation 37 Compound 313

Method: General Procedure 13
Starting material: Compound 213
Chromatography eluant: 10% ether in petroleum ether
¹H NMR δ 0.05 (m, 1 2H), 0.22 (m, 1H), 0.32 (m, 1H), 0.49 (m, 2H), 0.68 (s, 3H), 0.85 (s, 9H), 0.86 (s, 9H), 0.97 (s, 1H), 1.15 (d, 3H), 1.40-2.10 (m, 9H), 2.15-2.45 (m, 3H), 2.58 (dd, 1H), 2.87 (m, 2H), 3.42 (m, 1H), 4.22 (m, 1H), 4.53 (m, 1H), 4.94 (m, 1H), 5.00 (m, 1H), 5.37 (m, 1H), 5.54 (m, 2H), 5.90 (d, 1H), 6.44 (d, 1H).

### Preparation 38 Compound 314

Method: General Procedure 13
Starting material: Compound 214
Chromatography eluant: 10% ether in petroleum ether

### Preparation 39 Compound 315

Method: General Procedure 13
Starting material: Compound 213
Chromatography eluant: 10% ether in petroleum ether
¹H NMR δ 0.05 (m, 12H), 0.23 (m, 1H) 0.32 (m, 1H), 0.51 (m, 2H), 0.68 (s, 3H), 0.85 (s, 6H), 0.90 (s, 9H), 0.98 (m, 9H), 1.15 (d, 3H), 1.45-2.10 (m, 9H), 2.17-2.45 (m, 3H), 2.58 (dd, 1H), 2.87 (m 2H), 3.46 (m, 1H), 4.22 (m, 1H), 4.54 (m, 1H), 4.94 (m, 1H), 4.98 (m, 1H), 5.37 (m, 1H), 5.58 (m, 2H), 5.90 (d, 1H), 6.45 (d, 1H).

### Preparation 40 Compound 316

Method: General Procedure 13
Starting material: Compound 214
Chromatography eluant: 10% ether in petroleum ether.

### Preparation 41 Compound 401

Method: General Procedure 14
Starting material: Compound 201
Chromatography eluant: 10% ether in pentane
¹H NMR δ 0.06 (m, 12H), 0.71 (s, 3H), 0.86 (s, 9H), 0.87 (s, 9H), 1.19 (d, 3H), 1.56 (s, 6H), 1.15-2.70 (m, 14H), 2.82 (m, 1H), 3.80 (t, 1H), 4.00 (dd, 1H), 4.19 (m, 1H), 4.37 (m, 1H), 4.87 (m, 1H), 5.18 (m, 1H), 5.45 (m, 1H), 6.10 (d, 1H), 6.23 (d, 1H), 6.77 (m, 1H), 7.03 (m, 2H), 7.23 (t, 1H).

### Preparation 42 Compound 402

Method: General Procedure 14
Starting material: Compound 202
Chromatography eluant: 10% ether in pentane
¹H NMR δ 0.06 (m, 12H), 0.71 (s, 3H), 0.87 (s, 18H), 1.22 (d, 3H), 1.57 (s, 6H), 1.40-2.15 (m, 9H), 2.23 (m, 2H), 2.37 (m, 1H), 2.45 (dd, 1H), 2.61 (m, 1H), 2.82 (m, 1H), 3.66 (t, 1H), 4.01 (dd, 1H), 4.19 (m, 1H), 4.38 (m, 1H), 4.88 (m, 1H), 5.18 (m, 1H), 5.48 (m, 1H), 6.11 (d, 1H), 6.23 (d, 1H), 6.77 m, 1H), 6.95-7.10 (m, 2H), 7.24 (t, 1H).

### Preparation 43 Compound 403

Method: General Procedure 14
Starting material: Compound 203
Chromatography eluant: 10% ether in pentane
¹H NMR δ 0.06 (m, 12H), 0.69 (s, 3H), 0.87 (s, 18H), 1.18 (d, 3H), 1.55 (s, 6H), 1.15-2.50 (m, 14H), 2.80 (m, 1H), 2.86 (dd, 1H), 3.20 (dd, 1H), 4.18 (m, 1H), 4.38 (m, 1H), 4.87 (m, 1H), 5.19 (m, 1H), 5.41 (m, 1H), 6.09 (d, 1H), 6.22 (d, 1H), 7.15-7.30 (m, 3H), 7.46 (m, 1H).

### Preparation 44 Compound 404

Method: General Procedure 14
Starting material: Compound 204
Chromatography eluant: 10% ether in pentane
¹H NMR δ 0.05 (m, 12H), 0.66 (s, 3H), 0.86 (s, 9H), 0.87 (s, 9H), 1.21 (d, 3H), 1.56 (s, 6H), 1.30-2.50 (m, 14H), 2.78 (dd, 1H), 2.80 (m, 1H), 3.18 (dd, 1H), 4.18 (m, 1H), 4.37 (m, 1H), 4.86 (m, 1H), 5.18 (m, 1H), 5.44 (m, 1H), 6.08 (d, 1H), 6.22 (d, 1H), 7.15-7.30 (m, 3H), 7.47 (m, 1H).

### Preparation 45 Compound 405

Method: General Procedure 14
Starting material: Compound 305
Chromatography eluant: 10% ether in pentane
¹H NMR δ 0.05 (m, 12H), 0.67 (s, 3H), 0.87 (s, 9H), 0.88 (s, 9H), 1.01 (d, 3H), 1.18 (s, 6H), 0.80-2.27 (m, 18H), 2.35 (m, 1H), 2.45 (dd, 1H), 2.80 (m, 1H), 4.19 (m, 1H), 4.37 (m, 1H), 4.87 (m, 1H), 5.18 (m, 1H), 5.28 (m, 1H), 6.09 (d, 1H), 6.23 (d, 1H).

### Preparation 46 Compound 406

Method: General Procedure 14
Starting material: Compound 306
Chromatography eluant: 10% ether in pentane
¹H NMR δ 0.06 (m, 12H), 0.68 (s, 3H), 0.87 (s, 9H), 0.88 (s, 9H), 1.04 (d, 3H), 1.20 (s, 6H), 1.10-2.40 (m, 19H), 2.45 (d, 1H), 2.80 (m, 1H), 4.18 (m, 1H), 4.38 (m, 1H), 4.87 (m, 1H), 5,18 (m, 1H), 5.30 (m, 1H), 6.10 (d, 1H), 6.23 (d, 1H).

### Preparation 47 Compound 407

Method: General Procedure 14
Starting material: Compound 307
Chromatography eluant: 10% ether in pentane
¹H NMR δ 0.06 (m, 12H), 0.66 (s, 3H), 0.84 (t, 6H), 0.87 (s, 18H), 1.00 (d, 3H), 1.44 (q, 4H), 0.75-2.27 (m, 20H), 2.34 (m, 1H), 2.44 (dd, 1H), 2.80 (m, 1H), 4.19 (m, 1H), 4.38 (m, 1H), 4.87 (m, 1H), 5.18 (m, 1H), 5.27 (m, 1H), 6.09 (d, 1H), 6.22 (d, 1H).

### Preparation 48 Compound 408

Method: General Procedure 14
Starting material: Compound 308
Chromatography eluant: 10% ether in pentane
¹H NMR δ 0.06 (m, 12H), 0.68 (s, 3H), 0.87 (t, 6H), 0.87 (s, 9H), 0.88 (s, 9H), 1.02 (d, 3H), 1.44 (q, 4H), 1.15-2.50 (m, 22H), 2.80 (m, 1H), 4.19 (m, 1H), 4.38 (m, 1H), 4.87 (m, 1H), 5.18 (m, 1H), 5.29 (m, 1H), 6.09 (d, 1H), 6.23 (d, 1H).

### Preparation 49 Compound 409

Method: General Procedure 14
Starting material: Compound 309
Chromatography eluant: 5% ether in petroleum ether

### Preparation 50 Compound 410

Method: General Procedure 14
Starting material: Compound 310
Chromatography eluant: 5% ether in petroleum ether

### Preparation 51 Compound 411

Method: General Procedure 14
Starting material: Compound 311
Chromatography eluant: 5% ether in pentane
¹H NMR δ 0.05 (m, 12H), 0.66 (s, 3H), 0.85 (t, 6H), 0.85 (s, 9H), 0.86 (s, 9H), 1.15 (d, 3H), 1.10-1.92 (m, 12H), 1.99 (m, 1H), 2.18 (m, 2H), 2.33 (dd, 1H), 2.45 (dd, 1H), 2.77 (dd, 1H), 2.90 (m, 1H), 4.18 (m, 1H), 4.37 (m, 1H), 4.87 (m, 1H), 5.17 (m, 1H), 5.35 (m, 1H), 5.53 (d, 1H, J = 15.3 Hz), 5.58 (dd, 1H, J = 14.9 Hz and 8.0 Hz), 5.95-6.27 (m, 4H).

### Preparation 52 Compound 412

Method: General Procedure 14
Starting material: Compound 312
Chromatography eluant: 5% ether in pentane
¹H NMR δ 0.05 (m, 12H), 0.68 (s, 3H), 0.86 (s, 9H), 0.87 (s, 9H), 0.83-0.95 (t, 6H), 1.16 (d, 3H), 1.10-2.30 (m, 15H), 2.36 (dd, 1H), 2.44 (dd, 1H), 2.79 (m, 1H), 2.88 (m, 1H), 4.18 (m, 1H), 4.37 (m, 1H), 4.87 (m, 1H), 5.17 (m, 1H), 5.35 (m, 1H), 5.53 (d, 1H, J = 15.4 Hz), 5.64 (dd, 1H, J = 7.7 and 15.0 Hz), 5.92-6.25 (m, 4H).

### Preparation 53 Compound 413

Method: General Procedure 14
Starting material: Compound 313
Chromatography eluant: 10% ether in petroleum ether
¹H NMR δ 0.05 (m, 12H), 0.23 (m, 1H), 0.31 (m, 1H), 0.50 (m, 2H), 0.67 (s, 3H), 0.86 (s, 9H) 0.87 (s, 9H), 0.97 (s, 1H), 1.14 (d, 3H), 1.30-2.07 (m, 9H), 2.20 (m, 2H), 2.32 (m, 1H), 2.44 (dd, 1H), 2.79 (m, 1H), 2.89 (m, 1H), 3.42 (m, 1H), 4.19 (m, 1H), 4.37 (m, 1H), 4.87 (m, 1H), 5.17 (m, 1H), 5.35 (m, 1H), 5.54 (m, 2H), 6.08 (d, 1H), 6.22 (d, 1H).

### Preparation 54 Compound 414

Method: General Procedure 14
Starting material: Compound 314
Chromatography eluant: 10% ether in petroleum ether

### Preparation 55 Compound 415

Method: General Procedure 14
Starting material: Compound 315
Chromatography eluant: 10% ether in petroleum ether
¹H NMR δ 0.05 (m, 12H), 0.23 (m, 1H), 0.31 (m, 1H), 0.50 (m, 2H), 0.67 (s, 3H), 0.86 (s, 9H), 0.87 (s, 9H), 1.14 (d, 3H), 0.90-2.05 (m, 10H), 2.19 (m, 2H), 2.33 (m, 1H), 2.44 (dd, 1H), 2.79 (m, 1H), 2.89 (m, 1H), 3.46 (m, 1H), 4.18 (m, 1H), 4.36 (m, 1H), 4.87 (m, 1H), 5.17 (m, 1H), 5.34 (m, 1H), 5.57 (m, 2H), 6.08 (d, 1H), 6.22 (d, 1H).

### Preparation 56 Compound 416

Method: General Procedure 14
Starting material: Compound 316
Chromatography eluant: 10% ether in petroleum ether

### Examples

### Example 1: 1(S),3(R)-Dihydroxy-20(S)-(3-(1-hydroxy-1-methylethyl)phenoxymethyl)-9,10-seco-pregna-5(Z),-7(E),10(19),16-tetraene (Compound 101)

Method: General Procedure 15
Starting material: Compound 401
Chromatography eluant: 30% pentane in ethyl acetate.
¹H NMR δ 0.73 (s, 3H), 1.20 (d, 3H), 1.57 (s, 6H), 1.15-1.97 (m, 9H), 2.04 (m, 2H), 2.17-2.45 (m, 3H), 2.60 (m, 2H), 2.83 (dd, 1H), 3.81 (t, 1H), 4.00 (dd, 1H), 4.23 (m, 1H), 4.43 (m, 1H), 5.01 (m, 1H), 5.33 (m, 1H), 5.45 (m, 1H), 6,12 (d, 1H), 6.37 (d, 1H), 6.77 (m, 1H), 7.04 (m, 2H), 7.24 (t, 1H).

### Example 2: 1(S),3(R)-Dihydroxy-20(R)-(3-(1-hydroxy-1-methylethyl)phenoxymethyl)-9,10-seco-pregna-5(Z),7(E),-10(19),16-tetraene (Compound 102)

Method: General Procedure 15
Starting material: Compound 402
Chromatography eluant: 30% pentane in ethyl acetate.
¹H NMR δ 0.73 (s, 3H), 1.23 (d, 3H), 1.57 (s, 6H), 1.45-1.97 (m, 9H), 2.05 (m, 2H), 2.15-2.45 (m, 3H), 2.60 (m, 2H), 2.83 (m, 1H), 3.67 (t, 1H), 4.00 (dd, 1H), 4.23 (m, 1H), 4.43 (m, 1H), 5.02 (m, 1H), 5.34 (m, 1H), 5.49 (m, 1H), 6.12 (d, 1H), 6.37 (d, 1H), 6.77 (m, 1H), 7.04 (m, 2H), 7.24 (t, 1H).

### Example 3: 1(S),3(R)-Dihydroxy-20(S)-(3-(1-hydroxy-1-methylethyl)phenylthiomethyl)-9,10-seco-pregna-5(Z),-7(E),10(19),16-tetraene (Compound 103)

Method: General Procedure 15
Starting material: Compound 403
Chromatography eluant: 30% pentane in ethyl acetate.
¹H NMR δ 0.71 (s, 3H), 1.19 (d, 3H), 1.56 (s, 6H), 1.15-2.50 (m, 15H), 2.58 (dd, 1H), 2.81 (dd 1H), 2.87 (dd, 1H), 3.19 (dd, 1H), 4.23 (m, 1H), 4.43 (m, 1H), 5.00 (m, 1H), 5.33 (m, 1H), 5.42 (m, 1H), 6.11 (d, 1H), 6.36 (d, 1H), 7.15-7.30 (m, 3H), 7.46 (m, 1H).

### Example 4: 1(S),3(R)-Dihydroxy-20(R)-(3-(1-hydroxy-1-methylethyl)phenylthiomethyl)-9,10-seco-pregna-5(Z),7(E),-10(19),16-tetraene (Compound 104)

Method: General Procedure 15
Starting material: Compound 404
Chromatography eluant: 30% pentane in ethyl acetate.
¹H NMR δ 0.68 (s, 3H), 1.22 (d, 3H), 1.57 (s, 6H), 1.30-2.50 (m, 15H), 2.60 (dd, 1H), 2.80 (dd, 1H), 2.82 (m, 1H), 3.18 (dd, 1H), 4.23 (m, 1H), 4.44 (m, 1H), 5.00 (m, 1H), 5.33 (m, 1H), 5.46 (m, 1H), 6.10 (d, 1H), 6.36 (d, 1H), 7.15-7.30 (m, 3H), 7.48 (m, 1H).

### Example 5: 1(S),3(R)-Dihydroxy-20(R)-(4-hydroxy-4-methylpent-1-yl)-9,10-seco-pregna-5(Z),7(E),10(19),16-tetraene (Compound 105)

Method: General Procedure 15
Starting material: Compound 405
Chrornatography eluant: 40% pentane in ethyl acetate.
¹H NMR δ 0.69 (s, 3H), 1.02 (d, 3H), 1.19 (s, 6H), 0.80-2.40 (m, 21H), 2.60 (dd, 1H), 2.82 (m, 1H), 4.22 (m, 1H), 4.44 (m, 1H), 5.02 (m, 1H), 5.29 (m, 1H), 5.34 (m, 1H), 6.11 (d, 1H), 6.38 (d, 1H).

### Example 6: 1(S),3(R)-Dihydroxy-20(S)-(4-hydroxy-4-methylpent-1-yl)-9,10-seco-pregna-5(Z),7(E),10(19),16-tetraene (Compound 106)

Method: General Procedure 15
Starting material: Compound 406
Chromatography eluant: 30% pentane in ethyl acetate.
¹H NMR δ 0.70 (s, 3H), 1.05 (d, 3H), 1.21 (s, 6H), 1.15-2.40 (m, 21H), 2.60 (m, 1H), 2.82 (m, 1H), 4.24 (m, 1H), 4.42 (m, 1H), 5.01 (m, 1H), 5.33 (m, 2H), 6.10 (d, 1H), 6.37 (d, 1H).

### Example 7: 1(S),3(R)-Dihydroxy-20(R)-(5-ethyl-5-hydroxyhept-1-yl)-9,10-seco-pregna-5(Z),7(E),10(19),16-tetraene (Compound 107)

Method: General Procedure 15
Starting material: Compound 407
Chromatography eluant: 40% pentane in ethyl acetate.
¹H NMR δ 0.68 (s, 3H), 0.85 (t, 6H), 1.01 (d, 3H), 1.45 (q, 4H), 0.80-2.45 (m, 23H), 2.60 (m, 1H), 2.82 (m, 1H), 4.23 (m, 1H), 4.44 (m, 1H), 5.02 (m, 1H), 5.28 (m, 1H), 5.34 (m, 1H), 6.11 (d, 1H), 6.38 (d, 1H).

### Example 8: 1(S),3(R)-Dihydroxy-20(S)-(5-ethyl-5-hydroxy-hept-1-yl)-9,10-seco-pregna-5(Z),7(E),10(19),16-tetraene (Compound 108)

Method: General Procedure 15
Starting material: Compound 408
Chromatography eluant: 40% pentane in ethyl acetate.
¹H NMR δ 0.70 (s, 3H), 0.85 (t, 6H), 1.03 (d, 3H), 1.45 (q, 4H), 1.00-2.40 (m, 23H), 2.60 (dd, 1H), 2.81 (m, 1H), 4.23 (m, 1H), 4.43 (m, 1H), 5.01 (m, 1H), 5.31 (m, 1H), 5.33 (m, 1H), 6.11 (d, 1H), 6.37 (d, 1H).

### Example 9: 1(S),3(R)-Dihydroxy-20(R)-(5-hydroxy-5-methyl-hexa-1(E),3(E)-dien-1-yl)-9,10-seco-pregna--5(Z),7(E),10(19),16-tetraene (Compound 109)

Method: General Procedure 16
Starting material: Compound 409

### Example 10: 1(S),3(R)-Dihydroxy-20(S)-(5-hydroxy-5-methyl-hexa-1(E),3(E)-dien-1-yl)9,10-seco-pregna--5(Z),7(E),10(19),16-tetraene (Compound 110)

Method: General Procedure 15
Starting material: Compound 410

### Example 11: 1(S),3(R)-Dihydroxy-20(R)-(5-ethyl-5-hydroxyhepta-1(E),3(E)-dien-1-yl)-9,10-seco-pregna--5(Z),7(E),10(19),16-tetraene (Compound 111)

Method: General Procedure 16
Starting material: Compound 411
¹H NMR δ 0.68 (s, 3H), 0.86 (t, 6H), 1.16 (d, 3H), 1.56 (q, 4H), 1.35-2.43 (m, 14H), 2.59 (dd, 1H), 2.80 (dd, 1H), 2.91 (m, 1H), 4.23 (m, 1H), 4.43 (m, 1H), 5.00 (m, 1H), 5.33 (m, 1H), 5.37 (m, 1H), 5.55 (d, 1H, J = 15.4 Hz), 5.58 (dd, 1H, J = 14.9 Hz and 8.0 Hz), 5.95-6.25 (m, 3H), 6.36 (d, 1H).

### Example 12: 1(S),3(R)-Dihydroxy-20(S)-(5-ethyl-5-hydroxyhepta-1(E),3(E)-dien-1-yl)-9,10-seco-pregna-5(Z),7(E),10-(19),16-tetraene (Compound 112)

Method: General Procedure 16
Starting material: Compound 412
¹H NMR δ 0.71 (s, 3H), 0.86 (t, 6H), 1.17 (d, 3H), 0.83-2.45 (m, 18H), 2.60 (dd, 1H), 2.80 (m, 1H), 2.89 (m, 1H), 4.23 (m, 1H), 4.44 (m, 1H), 5.01 (m, 1H), 5.33 (m, 1H), 5.37 (m, 1H), 5.55 (d, 1H, J = 15.3 Hz), 5.64 (dd, 1H, J = 7.7 and 15.0 Hz), 6.01 (dd, 1H, J = 10.3 and 15.0 Hz), 6.11 (d, 1H), 6.18 (dd, 1H, J = 10.3 and 15.3 Hz), 6.37 (d, 1H).

### Example 13: 1(S),3(R)-Dihydroxy-20(R)-(3-cyclo-propyl-3--hydroxyprop-1(E)-en-1-yl)-9,10-seco-pregna-5(Z),7(E),-10(19),16-tetraene (24(S)-isomer) (Compound 113)

Method: General Procedure 16
Starting material: Compound 413
¹H NMR δ 0.23 (m, 1H), 0.32 (m, 1H), 0.51 (m, 2H), 0.70 (s, 3H), 0.99 (m, 1H), 1.15 (d, 3H), 1.10-2.40 (m, 14H), 2.59 (dd, 1H), 2.80 (dd, 1H), 2.90 (m, 1H), 3.42 (m, 1H), 4.23 (m, 1H), 4.43 (m, 1H), 5.00 (m, 1H), 5.33 (m; 1H), 5.37 (m, 1H), 5.55 (m, 2H), 6.10 (d, 1H), 6.36 (d, 1H).

### Example 14: 1(S),3(R)-Dihydroxy-20(S)-(3-cyclo-propyl-3--hydroxyprop-1(E)-en-1-yl)-9,10-seco-pregna--5(Z),7(E),10(19),16-tetraene (24(S)-isomer) (Compound 114)

Method: General Procedure 16
Starting material: Compound 414

### Example 15: 1(S),3(R)-Dihydroxy-20(R)-(3-cyclo-propyl-3--hydroxyprop-1(E)-en-1-yl)-9,10-seco-pregna--5(Z),7(E),10(19),16-tetraene (24(R)-isomer) (Compound 115)

Method: General Procedure 16
Starting material: Compound 415
¹H NMR δ 0.23 (m, 1H), 0.32 (m, 1H), 0.51 (m, 2H), 0.70 (s, 3H), 0.99 (m, 1H), 1.15 (d, 3H), 1.12-2.45 (m, 14H), 2.60 (bd, 1H), 2.80 (m, 1H), 2.90 (m, 1H), 3.46 (m, 1H), 4.23 (m, 1H), 4.44 (m, 1H), 5.00 (m, 1H), 5.33 (m, 1H), 5.36 (m, 1H), 5.58 (m, 2H), 6.10 (d, 1H), 6.36 (d, 1H).

### Example 16: 1(S),3(R)-Dihydroxy-20(S)-(3-cyclo-propyl-3--hydroxyprop-1(E)-en-1-yl)-9,10-seco-pregna--5(Z),7(E),10(19),16-tetraene (24(R)-isomer) (Compound 116)

Method: General Procedure 16
Starting material: Compound 416

### Example 17 Capsules containing Compound 111

Compound 111 was dissolved in arachis oil to a final concentration of 1 µg of Compound 111/ml oil. 10 Parts by weight of gelatine, 5 parts by weight glycerine, 0.08 parts by weight potassium sorbate, and 14 parts by weight distilled water were mixed together with heating and formed into soft gelatine capsules. These were then filled each with 100 µl of Compound 111 in oil solution, such that each capsule contained 0.1 µg of Compound 111.

### Example 18 Dermatological Cream Containing Compound 111

In 1 g almond oil was dissolved 0.05 mg of Compound 111. To this solution was added 40 g of mineral oil and 20 g of self-emulsifying beeswax. The mixture was heated to liquify. After the addition of 40 ml hot water, the mixture was mixed well. The resulting cream contains approximately 0.5 µg of Compound 111 per gram of cream.

## Claims

1. A compound of the formula I 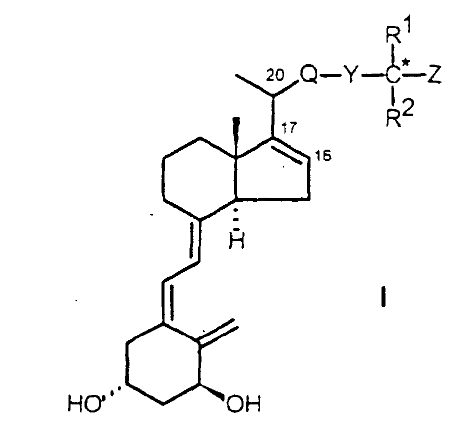 in which formula Q is methylene, ethylene, tri-, tetra- or pentamethylene, -CH=CH-, -CH=CH-CH=CH-, -CH=CH-C≡C-, -CH=CH-CH₂-, -C≡C-, -C≡C-CH₂-, -CH(R)-(CH₂)₂-, -CH(R)-CH=CH-, or -CH(R)-C≡C- in which R is C₁-C₃ alkyl; Y is either a single bond, a carbonyl group or a methylene, ethylene, -CH(OH)-, -O-(C₆H₄)- (ortho, meta, para) or -S-(C₆H₄)- (ortho, meta, para) diradical; R¹ and R² , which may be the same or different, stand for hydrogen or a C₁-C₆ hydrocarbyl radical; or R¹ and R², when taken together with the carbon atom (starred in formula I) bearing the group Z, can form a C₃-C₆ carbocycilic ring; and Z is hydrogen or hydroxy; with the proviso that when, at the same time, Q is ethylene, Y stands for methylene, carbonyl or -CH(OH)-, R¹ and R² are methyl, and Z is hydroxy, then the configuration at C-20 cannot be R.

2. A diastereoisomer of a compound according to claim 1, in pure form; or a mixture of diastereoisomers of a compound according to claim 1.

3. A compound according to claim 1 which is:
a) 1(S),3(R)-Dihydroxy-20(S)-(3-(1-hydroxy-1-methylethyl)phenoxymethyl)-9,10-secopregna-5(Z),7(E),10(19),16-tetraene or the corresponding 20(R) isomer,
b) 1(S),3(R)-Dihydroxy-20(S)-(3-(1-hydroxy-1-methylethyl)phenylthiomethyl)-9,10-secopregna-5(Z),7(E),10(19),16-tetraene or the corresponding 20(R) isomer,
c) 1(S),3(R)-Dihydroxy-20(S)-(4-hydroxy-4-methylpent-1-yl)-9,10-secopregna -5(Z),7(E),10(19),16-tetraene,
d) 1(S),3(R)-Dihydroxy-20(R)-(5-ethyl-5-hydroxyhept-1-yl)-9,10-secopregna-5(Z),7(E),10(19),16-tetraene or the corresponding 20(S) isomer,
e) 1(S),3(R)-Dihydroxy-20(R)-(5-ethyl-5-hydroxyhepta-1(E),3(E)-dien-1-yl)-9,10-secopregna-5(Z),7(E),10(19),16-tetraene or the corresponding 20(S) isomer,
f) 1(S),3(R)-Dihydroxy-20(R)-(3-cyclopropyl-3-hydroxyprop-1(E)-en-1-yl)-9,10-secopregna-5(Z),7(E),10(19),16-tetraene (24(S) isomer) or the corresponding 24(R) isomer.

4. A method for producing a compound of formula I of claim 1 by which:
a) the side chain attached to C-20 in compound I is elaborated from the 20(S) and 20(R) isomers of 1(S),3(R)-bis-(tert-butyldimethylsilyloxy)-20-p-toluenesulfonyloxymethyl-9,10-secopregna-5(E),7(E),10(19),16-tetraene, either
(i) by reaction with a side chain building block H-X-R³ (X is 0 or S, R³ is -C₆H₄-CR¹R²Z¹ (meta), and Z¹ is hydroxy or protected hydroxyl) in the presence of a base (e.g. NaH) in a solvent (e.g. DMF), or
(ii) by reaction with a Grignard reagent R⁴-Mg-Hal (R⁴ is CH₂-(CH₂)ₙ-CR¹R²Z¹, n is 2, 3 or 4, Z¹ is as defined above, and Hal is Cl or Br) in the presence of Li₂CuCl₄ in a solvent (e.g. THF), and
b) the compound from step (a) above is optionally (i) separated from diastereoisomers (e.g. by chromatography), (ii) subjected to a triplet-sensitized photoisomerisation to the 5(Z) isomer, (iii) desilylated (e.g. with tetrabutylammonium fluoride, and (iv) otherwise deprotected; the order of these options being arbitrary.

5. A method of producing a compound of formula I of claim 1 by which:
a) the side chain attached to C-20 in compound I is elaborated from the 20(S) and 20(R) isomers of 1(S),3(R)-bis-(tert-butyldimethylsilyloxy)-20-formyl-9,10-secopregna-5(E),7(E),10(19),16-tetraene, either
(i) by reaction with a Wittig-type reagent
(e.g. (EtO)₂P(O)-CH(Li)-CH=CH-COOCH₃) followed by reaction of the resulting ester with an organometallic reagent (e.g. R¹Li), or
(ii) by reaction with a Wittig-type reagent 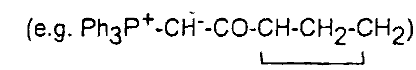 followed by reaction of the resulting ketone with a reducing agent (e.g. NaBH₄) in the presence of CeCl₃ , and
b) the compound from step (a) above is optionally (i) separated from diastereoisorners (e.g. by chromatography), (ii) subjected to a triplet-sensitized photoisomerisation to the 5(Z) isomer, (iii) desilylated (e.g. with tetrabutylammonium fluoride), and (iv) otherwise deprotected; the order of these options being arbitrary.

6. An intermediate for the synthesis of compounds of formula I and analogues thereof which is:
a) 1(S),3(R)-bis-(tert-butyldimethylsilyloxy)-20(S)-hydroxymethyl-9,10-seco-pregna-5(E),7(E),10(19),16-tetraene or the corresponding 20(R) isomer,
b) 1(S),3(R)-bis-(tert-butyldimethylsilyloxy)-20(S)-p-toluenesulfonyloxymethyl-9,10-secopregna-5(E),7(E),10(19),16-tetraene or the corresponding 20(R) isomer,
c) 1(S),3(E)-bis-(tert-butyldimethylsilyloxy)-20(S)-formyl-9,10-secopregna-5(E),7(E),10(19),16-tetraene orthe corresponding 20(R) isomer.

7. A pharmaceutical composition containing an effective amount of one or more of the compounds of claims 1-3, together with pharmaceutically acceptable, non-toxic carriers and/or auxiliary agents.

8. A pharmaceutical composition according to claim 7 in dosage unit form containing from 0.1 ppm to 0.1% by weight of a compound of formula I.

9. The use of a compound of any one of claims 1-3 in the manufacture of a medicament for the treatment or prophylaxis of hyperparathyroidism, particularly secondary hyperparathyroidism associated with renal failure, of diseases **characterized by** abnormal cell differentiation and/or cell proliferation such as cancer, leukemia, myelofibrosis, and psoriasis, of a number of disease states including diabetes mellitus, hypertension, acne, alopecia, skin ageing, AIDS, neurodegenerative disorders such as Alzheimer's disease, host versus graft reactions, rejection of transplants, inflammatory diseases such as rheumatoid arthritis and asthma, for prevention and/or treatment of steroid Induced skin atrophy, and for promoting osteogenesis and treating osteoporosis.

## Patentansprüche

1. Verbindung der Formel I 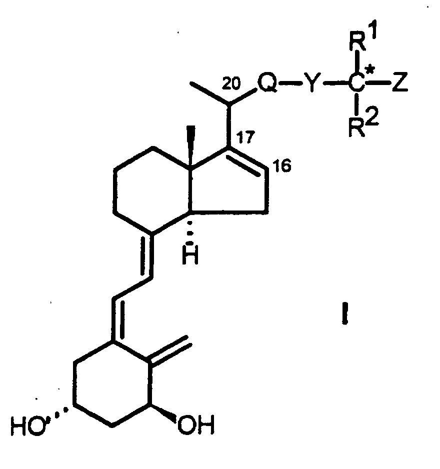 worin Q für Methylen, Ethylen, Tri-, Tetra- oder Pentamethylen, -CH=CH-, -CH=CH-CH=CH-, -CH=CH-C≡C-, -CH=CH-CH₂-, -C≡C-, -C≡C-CH₂-, -CH(R)-(CH₂)₂-, -CH(R)-CH=CH- oder -CH(R)-C≡C- steht, worin R C₁-C₃-Alkyl ist; Y entweder für eine Einfachbindung, eine Carbonylgruppe oder einen zweiwertigen Methylen-Rest, Ethylen-Rest, -CH(OH)-Rest, -O-(C₆H₄)-Rest (ortho, meta, para) oder -S-(C₆H₄)-Rest (ortho, meta, para) steht; R¹ und R², die gleich oder verschieden sein können, für Wasserstoff oder einen C₁-C₆-Kohlenwasserstoffrest stehen; oder R¹ und R² zusammen mit dem die Gruppe Z tragenden Kohlenstoffatom (in Formel I mit * gekennzeichnet) einen C₃-C₆carbocyclischen Ring bilden können; und Z Wasserstoff oder Hydroxy ist; mit der Maßgabe, dass die Konfiguration am C-20 nicht R sein kann, falls Q Ethylen, Y Methylen, Carbonyl oder -CH(OH)-, R¹ und R² Methyl und Z Hydroxy sind.

2. Diastereomer einer Verbindung nach Anspruch 1 in reiner Form; oder ein Gemisch von Diastereomeren einer Verbindung nach Anspruch 1.

3. Verbindung nach Anspruch 1, nämlich:
a) 1(S),3(R)-Dihydroxy-20(S)-(3-(1-hydroxy-1-methylethyl)phenoxymethyl)-9,10-secopregna-5(Z),7(E),10(19),16-tetraen oder das entsprechende 20(R)-Isomer,
b) 1(S),3(R)-Dihydroxy-20(S)-(3-(1-hydroxy-1-methylethyl)phenylthiomethyl)-9,10-secopregna-5(Z),7(E),10(19),16-tetraen oder das entsprechende 20(R)-Isomer,
c) 1(S),3(R)-Dihydroxy-20(S)-(4-hydroxy-4-methylpent-1-yl)-9,10-secopregna-5(Z),7(E),10(19),16-tetraen,
d) 1(S),3(R)-Dihydroxy-20(R)-(5-ethyl-5-hydroxyhept-1-yl)-9,10-secopregna-5(Z),7(E),10(19),16-tetraen oder das entsprechende 20(S)-Isomer,
e) 1(S),3(R)-Dihydroxy-20(R)-(5-ethyl-5-hydroxyhept-1(E),3(E)-dien-1-yl)-9,10-secopregna-5(Z),7(E),10(19),16-tetraen oder das entsprechende 20(S)-Isomer,
f) 1(S),3(R)-Dihydroxy-20(R)-(3-cyclopropyl-3-hydroxyprop-1(E)-en-1-yl)-9,10-secopregna-5(Z),7(E),10(19),16-tetraen (24(S)-Isomer) oder das entsprechende 24(R)-Isomer.

4. Verfahren zur Herstellung einer Verbindung nach Formel I nach Anspruch 1, wobei man:
a) die in Verbindung I an C-20 gebundene Seitenkette aus den 20(S)-und 20(R)-Isomeren von 1(S),3(R)-Bis-(tert-butyldimethylsilyloxy)-20-ptoluolsulfonyloxymethyl-9,10-secopregna-5(E),7(E),10(19),16-tetraen bildet, und zwar entweder
(i) durch Umsetzung mit einem Seitenkettenbaustein H-X-R³ (X ist O oder S, R³ ist -C₆H₄-CR¹R²Z¹ (Meta) und Z¹ ist Hydroxy oder geschütztes Hydroxy) im Beisein einer Base (z.B. NaH) in einem Lösungsmittel (z.B. DMF), oder
(ii) durch Umsetzung mit einem Grignard-Reagens R⁴-Mg-Hal (R⁴ = CH₂-(CH₂)ₙ-CR¹R²Z¹, n ist 2, 3, oder 4, Z¹ ist wie oben definiert, und Hal ist Cl oder Br) im Beisein von Li₂CuCl₄ in einem Lösungsmittel (z.B. THF), und
b) die Verbindung aus obigem Schritt (a) gegebenenfalls (i) von Diastereomeren (z.B. durch Chromatographie) abtrennt, (ii) einer Triplet-sensitivierten Photoisomerisation zum 5(Z)-Isomer unterwirft, (iii) desilyliert (z.B. mit Tetrabutylammoniumfluorid), und (iv) auf andere Weise Schutzgruppen entfernt; wobei die Reihenfolge dieser Optionen beliebig ist.

5. Verfahren zur Herstellung einer Verbindung der Formel I nach Anspruch 1, wobei man:
a) die in Verbindung I an C-20 gebundene Seitenkette aus den 20(S)-und 20(R)-Isomeren von 1(S),3(R)-Bis-(tert-butyldimethylsilyloxy)-20-formyl-9,10-secopregna-5(E),7(E)10(19),16-tetraen bildet, und zwar entweder
(i) durch Umsetzung mit einem Reagens vom Wittig-Typ (z.B. (EtO)₂P(O)-CH(Li)-CH=CH-COOCH₃) und anschließender Umsetzung des resultierenden Esters mit einem organometallischen Reagens (z.B. R¹Li), oder
(ii) durch Umsetzung mit einem Reagens vom Wittig-Typ (z.B. Ph₃P⁺-CH⁻-CO-CH-CH₂-CH₂) und anschließender Umsetzung des resultierenden Ketons mit einem reduzierenden Agens (z.B. NaBH₄) im Beisein von CeCl₃, und
b) die Verbindung aus obigem Schritt (a) gegebenenfalls (i) von Diastereomeren (z.B. durch Chromatographie) abtrennt, (ii) einer Triplet-sensitivierten Photoisomerisation zum 5(Z)-Isomer unterwirft, (iii) desilyliert (z.B. mit Tetrabutylammoniumfluorid), und (iv) auf andere Weise Schutzgruppen entfernt; wobei die Reihenfolge dieser Optionen beliebig ist.

6. Zwischenprodukt für die Synthese von Verbindungen der Formel I und Analoga davon, nämlich:
a) 1(S),3(R)-Bis-(tert-butyldimethylsilyloxy)-20(S)-hydoxymethyl-9,10-secopregna-5(E),7(E),10(19),16-tetraen oder das entsprechende 20(R)-Isomer,
b) 1(S),3(R)-Bis-(tert-butyldimethylsilyloxy)-20(S)-ptoluolsulfonyloxymethyl-9,10-secopregna-5(E),7(E),10(19),16-tetraen oder das entsprechende 20(R)-Isomer,
c) 1(S),3(E)-Bis-(tert-butyldimethylsilyloxy)-20(S)-formyl-9,10-secopregna-5(E),7(E),10(19),16-tetraen oder das entsprechende 20(R)-Isomer.

7. Pharmazeutische Zusammensetzung, die eine wirksame Menge einer oder mehrerer Verbindungen der Ansprüche 1 bis 3 zusammen mit pharmazeutisch verträglichen, nichttoxischen Trägern und/oder Hilfsmitteln enthält.

8. Pharmazeutische Zusammensetzung nach Anspruch 7 in Form einer Dosierungseinheit, die 0,1 Gew.-ppm bis 0,1 Gew.-% einer Verbindung der Formel I enthält.

9. Verwendung einer Verbindung nach einem der Ansprüche 1 bis 3 bei der Herstellung eines Medikaments zur Behandlung oder Prophylaxe von Hyperparathyroidismus, insbesondere mit Nierenversagen einhergehendem sekundären Hyperparathyroidismus, von Erkrankungen, die durch eine anormale Zelldifferenzierung und/oder Zellproliferation gekennzeichnet sind, wie Krebs, Leukämie, Myelofibrose und Psoriasis, von einer Reihe von Erkrankungszuständen, zu denen Diabetes mellitus, Hypertonie, Akne, Alopezie, Hautalterung, AIDS, neurodegenerative Erkrankungen wie die Alzheimer'sche Erkrankung, Wirt-gegen-Transplantat-Reaktionen, Transplantatabstoßung, entzündliche Erkrankungen wie rheumatoide Arthritis und Asthma gehören, zur Prävention und/oder Behandlung steroidinduzierter Hautatrophie und zur Förderung der Osteogenese und zur Behandlung von Osteoporose.

## Revendications

1. Composé de formule 1 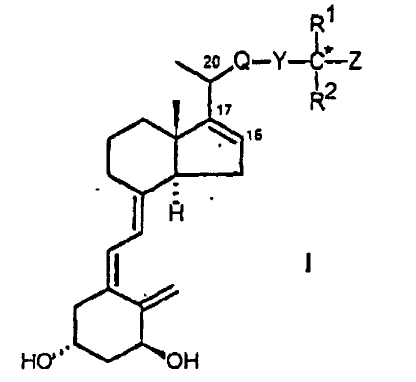 dans laquelle Q est un fragment méthylène, éthylène, tri-, tétra- ou pentaméthylène, -CH=CH-, -CH=CH-CH=CH-, -CH=CH-C≡C-, -CH=CH-CH₂-, -C≡C-, -C≡C-CH₂-, -CH(R)-(CH₂)₂-, -CH(R)-CH=CH- ou -CH(R)-C≡C-, où R est un alkyle en C₁-C₃; Y est une liaison simple, un groupe carbonyle ou un fragment divalent méthylène, éthylène, -CH(OH)-, -O-(C₆H₄)- (ortho, méta ou para) ou -S-(C₆H₄)- (ortho, méta ou para) ; R¹ et R², qui peuvent être identiques ou différents, représentent un hydrogène ou un reste hydrocarbyle en C₁-C₆ ; ou bien R¹ et R² peuvent constituer, avec l'atome de carbone (affecté d'un astérisque dans la formule I) portant le groupe Z, un cycle carbocyclique en C₃-C₆ ; et Z est un hydrogène ou un hydroxy ; sous réserve que dans le cas où, en même temps, Q est un éthylène, Y représente un méthylène, un carbonyle ou -CH(OH)-, R¹ et R² sont un méthyle et Z est un hydroxyle, alors la configuration en C-20 ne peut pas être R.

2. Diastéréoisomère d'un composé selon la revendication 1, sous forme pure ; ou mélange de diastéréoisomères d'un composé selon la revendication 1.

3. Composé selon la revendication 1, qui est :
a) le 1(S),3(R)-dihydroxy-20(S)-(3-(1-hydroxy-1-méthyléthyl)phénoxyméthyl)-9,10-sécoprégna-5(Z),7(E),10(19),16-tétraène ou l'isomère 20(R) correspondant,
b) le 1(S),3(R)-dihydroxy-20(S)-(3-(1-hydroxy-1-méthyléthyl)phénylthiométhyl)-9,10-sécoprégna-5(Z),7(E),10(19),16-tétraène ou l'isomère 20(R) correspondant,
c) le 1(S),3(R)-dihydroxy-20(S)-(4-hydroxy-4-méthylpent-1-yl)-9,10-sécoprégna-5(Z),7(E),10(19),16-tétraène,
d) le 1(S),3(R)-dihydroxy-20(R)-(5-éthyl-5-hydroxyhept-1-yl)-9,10-sécoprégna-5(Z),7(E),10(19),16-tétraène ou l'isomère 20(S) correspondant,
e) le 1(S),3(R)-dihydroxy-20(R)-(5-éthyl-5-hydroxyhepta-1(E),3(E)-dién-1-yl)-9,10-sécoprégna-5(Z),7(E),10(19),16-tétraène ou l'isomère 20(S) correspondant,
f) le 1(S),3(R)-dihydroxy-20(R)-(3-cyclopropyl-3-hydroxyprop-1(E)-én-1-yl)-9,10-sécoprégna-5(Z),7(E),10(19),16-tétraène (isomère 24(S))
ou l'isomère 24(R) correspondant.

4. Méthode pour la production d'un composé de formule I selon la revendication 1, par laquelle :
a) on élabore la chaîne latérale attachée au C-20 dans le composé I à partir des isomères 20(S) et 20(R) du 1(S),3(R)-bis(tert-butyldiméthylsilyloxy)-20-p-toluènesulfonyloxyméthyl-9,10-sécoprégna-5(E),7(E),10(19),16-tétraène,
(i) soit par une réaction avec un bloc de construction de la chaîne latérale H-X-R³ (X est O ou S, R³ est -C₆H₄-CR¹R²Z¹ (méta) et Z¹ est un hydroxyle ou un hydroxyle protégé) en présence d'une base (par exemple NaH) dans un solvant (par exemple le DMF),
(ii) soit par une réaction avec un réactif de Grignard R⁴-Mg-Hal (R⁴ est CH₂-(CH₂)ₙ-CR¹R²Z¹, n est égal à 2, 3 ou 4, Z¹ est tel que défini ci-dessus et Hal est Cl ou Br) en présence de Li₂CuCl₄ dans un solvant (par exemple THF) et
b) de façon optionnelle (i) on sépare le composé de l'étape (a) ci-dessus des diastéréoisomères (par exemple par chromatographie), (ii) on le soumet à une photoisomérisation sensibilisée par un triplet donnant l'isomère 5(Z), (iii) on le désilyle (par exemple avec le fluorure de tétrabutylammonium) et (iv) on le déprotège d'une autre façon ; l'ordre de ces options étant arbitraire.

5. Méthode de production d'un composé de formule I selon la revendication 1, par laquelle :
a) on élabore la chaîne latérale attachée au C-20 dans le composé I à partir des isomères 20(S) et 20(R) du 1(S),3(R)-bis(tert-bulyldiméthylsilyloxy)-20-formyl-9,10-sécoprégna-5(E),7(E),10(19),16-tétraène,
(i) soit par une réaction avec un réactif du type Wittig (par exemple (EtO)₂P(O)-CH(Li)-CH=CH-COOCH₃) suivie d'une réaction de l'ester obtenu avec un réactif organométallique (par exemple R¹Li),
(ii) soit par une réaction avec un réactif du type Wittig, par exemple 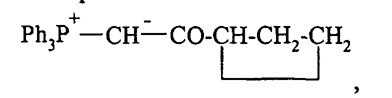 suivie d'une réaction de la cétone obtenue avec un agent réducteur (par exemple NaBH₄) en présence de CeCl₃ et
b) de façon optionnelle (i) on sépare le composé de l'étape (a) ci-dessus des diastéréoisomères (par exemple par chromatographie), (ii) on le soumet à une photoisomérisation sensibilisée par un triplet donnant l'isomère 5(Z), (iii) on le désilyle (par exemple avec le fluorure de tétrabutylammonium) et (iv) on le déprotège d'une autre façon ; l'ordre de ces options étant arbitraire.

6. Intermédiaire pour la synthèse de composés de formule I et de leurs analogues qui est :
a) le 1(S),3(R)-bis(tert-butyldiméthylsilyloxy)-20(S)-hydroxyméthyl-9,10-sécoprégna-5(E),7(E),10(19),16-tétraène ou l'isomère 20(R) correspondant,
b) le 1(S),3(R)-bis(tert-butyldiméthylsilyloxy)-20(S)-p-toluènesulfonyloxyméthyl-9,10-sécoprégna-5(E),7(E),10(19),16-tétraène ou l'isomère 20(R) correspondant,
c) le 1(S),3(E)-bis(tert-butyldiméthylsilyloxy)-20(S)-formyl-9,10-sécoprégna-5(E),7(E),10(19),16-tétraène ou l'isomère 20(R) correspondant.

7. Composition pharmaceutique contenant une quantité efficace d'un ou plusieurs des composés des revendications 1 à 3, en combinaison avec des supports et/ou des agents auxiliaires, non-toxiques, pharmaceutiquement acceptables.

8. Composition pharmaceutique selon la revendication 7, sous la forme de doses unitaires contenant de 0,1 ppm à 0,1 % en masse d'un composé de formule I.

9. Utilisation d'un composé selon l'une quelconque des revendications 1 à 3, pour la fabrication d'un médicament pour le traitement ou la prophylaxie d'une hyperparathyroïdie, en particulier d'une hyperparathyroïdie secondaire associée à une insuffisance rénale, de maladies **se caractérisant par** une différenciation cellulaire anormale et/ou une prolifération cellulaire comme le cancer, la leucémie, la myélofibrose, et le psoriasis, et de divers états pathologiques incluant le diabète, l'hypertension, l'acné, l'alopécie, le vieillissement de la peau, le SIDA, les troubles neurodégénératifs tels que la maladie d'Alzheimer, les réactions hôte contre greffe, le rejet de greffe, les maladies inflammatoires comme la polyarthrite rhumatoïde et l'asthme, pour la prévention et/ou le traitement d'une atrophie cutanée induite par les stéroïdes et pour favoriser l'ostéogenèse et traiter l'ostéoporose.
